(19) Europäisches Patentamt
European Patent Office
Office européen des brevets

(11) Numéro de publication: **0 292 348 B1**

(12) # FASCICULE DE BREVET EUROPEEN

(45) Date de publication du fascicule du brevet: **19.09.90**

(21) Numéro de dépôt: **88401062.0**

(22) Date de dépôt: **29.04.88**

(51) Int. Cl.⁵: **C07D 307/92**, C07D 333/74, C07D 235/02, C07D 209/60, C07D 263/60, C07D 277/60, A61K 31/335, A61K 31/38, A61K 31/395 // C07C215/86, C07C211/57, C07C323/38

(54) **Dérivés hétérocycliques polycycliques, leur procédé de préparation et leur utilisation en médecine humaine et vétérinaire.**

(30) Priorité: **30.04.87 FR 8706152**

(43) Date de publication de la demande: **23.11.88 Bulletin 88/47**

(45) Mention de la délivrance du brevet: **19.09.90 Bulletin 90/38**

(84) Etats contractants désignés: **AT BE CH DE ES FR GB GR IT LI NL SE**

(56) Documents cités:
**EP-A- 0 070 531**
**FR-A- 2 488 890**
**GB-A- 2 164 648**

**CHEMICAL ABSTRACTS, vol. 52, no. 16, 25 aôut 1958, colonne 13694, résumé no. 13694f, Columbus, Ohio, US; M.J. SCHLATTER:"Abnormal products from the Tert-butylation of P-cymene"**

(73) Titulaire: **CENTRE INTERNATIONAL DE RECHERCHES DERMATOLOGIQUES C.I.R.D. Groupement d'Intérêt Economique dit:, Sophia Antipolis, F-06560 Valbonne(FR)**

(72) Inventeur: **Shroot, Braham Villa 35, Hameaux de Val Bosquet Chemin de Val Bosquet, F-06600 Antibes(FR)**
Inventeur: **Eustache, Jacques, 42, avenue Alphonse Daudet, F-06130 Grasse(FR)**
Inventeur: **Bernardon, Jean-Michel, 21, Chemin Plan Bergier-Le Rouret, F-06650 Nice(FR)**
Inventeur: **Nedoncelle, Philippe, 10, boulevard Emile Zola, F-06130 Grasse(FR)**

(74) Mandataire: **Stalla-Bourdillon, Bernard et al, CABINET NONY & CIE 29, rue Cambacérès, F-75008 Paris(FR)**

EP 0 292 348 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet des dérivés hétérocycliques polycycliques, leur procédé de préparation et leur utilisation en médecine humaine et vétérinaire et en cosmétique.

Ces dérivés hétérocyliques trouvent une application dans le traitement topique et systémique des affections dermatologiques liées à un désordre de la kératinisation (différenciation-prolifération) et d'affections dermatologiques, ou autres, à composante inflammatoire et/ou immunoallergique et dans le traitement des maladies de dégénérescence du tissu conjonctif et présentent une activité antitumorale. En outre ces dérivates peuvent être utilisés dans le traitement de l'atopie, quelle soit cutanée ou respiratoire et du psoriasis rhumatoïde.

Enfin, ils trouvent une application dans le domaine ophtalmologique notamment dans le traitement des cornéopathies.

L'état de la technique concernant des dérivés hétérocycliques aromatiques ayant des propriétés similaires est représenté par le brevet GB 2 164 648.

Les dérivés hétérocycliques polycycliques selon l'invention peuvent être représentés par la formule générale suivante:

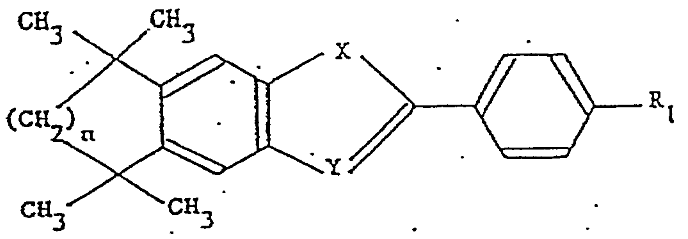

(I)

dans laquelle:

n est 1 ou 2

$R_1$ représente:

(i) un radical alkyle inférieur,

(ii) $-CH_2OH$

ou (iii) $-\underset{\underset{O}{\|}}{C}-R_2$

$R_2$ représentant:

(a) un atome d'hydrogène,

(b) le radical $-N\begin{smallmatrix} r' \\ r'' \end{smallmatrix}$

ou (c) le radical $-OR_3$, $R_3$ représentant un atome d'hydrogène, un radical alkyle ayant de 1 à 20 atomes de carbone, un radical mono ou polyhydroxyalkyle,un radical aryle ou aralkyle éventuellement substitué(s) ou un reste d'un sucre ou encore représenté le radical:

$$-(CH_2)_p-N\begin{smallmatrix} r' \\ r'' \end{smallmatrix}$$

p étant 1, 2 ou 3,

p étant 1, 2 ou 3, de chlorhydrates, de bromydrates ou de citrates lorsqu'ils comportent au moins une fonction amine.

Parmi les composés de formule (I), on peut notamment citer les suivants:

- l'ester méthylique de l'acide p-(5,6,7,8-tétrahydro-5,5,8,8-tétraméthyl-2-naphto[ 2,3-b ]furyl) benzoïque,

- l'acide p-(5,6,7,8-tétrahydro-5,5,8,8-tétraméthyl-2- naphto [ 2,3-b ]furyl ) benzoïque,
- l'ester méthylique de l'acide p-(5,6,7,8-tétrahydro-5,5,8,8-tétraméthyl-2-naphto[ 2,3-b ]thiényl) benzoïque,
- l'acide p-(5,6,7,8-tétrahydro-5,5,8,8-tétraméthyl-2-naphto [ 2,3-b ]thiényl) benzoïque,
- l'acide p-(5,6,7,8-tétrahydro-5,5,8,8-tétraméthyl-2-1H-benz [ f ]indolyl) benzoïque,
- l'ester méthylique de l'acide p-(5,6,7,8-tétrahydro-5,5,8,8-tétraméthyl-2-1H-benz[ f ]indolyl) benzoïque,
- l'ester méthylique de l'acide p-(1-méthyl-5,6,7,8-tétrahydro-5,5,8,8-tétraméthyl-2-benz[ f ]indolyl) benzoïque,
- l'acide p-(1-méthyl 5,6,7,8-tétrahydro-5,5,8,8-tétraméthyl-2-benz[ f ]indolyl) benzoïque,
- l'ester méthylique de l'acide p-(5,6,7,8-tétrahydro-5,5,8,8-tétraméthyl-2 napht[ 2,3-d ]oxazolyl) benzoïque,
- l'acide p-(5,6,7,8-tétrahydro-5,5,8,8-tétraméthyl-2-napht [ 2,3-d ]oxazolyl) benzoïque,
- l'alcool p-(5,6,7,8-tétrahydro-5,5,8,8-tétraméthyl-2-napht [ 2,3-d ]oxazolyl) benzylique,
- l'aldéhyde p-(5,6,7,8-tétrahydro-5,5,8,8-tétraméthyl-2-napht [ 2,3-d ]oxazolyl) benzoïque,
- l'éthylamide de l'acide p-(5,6,7,8-tétrahydro-5,5,8,8-tétraméthyl-2-napht[ 2,3-d ]oxazolyl benzoïque,
- le morpholide de l'acide p-(5,6,7,8-tétrahydro-5,5,8,8-tétraméthyl-2-napht[ 2,3-d ]oxazolyl) benzoïque,
- l'ester 2-hydroxyéthylique de l'acide p-(5,6,7,8-tétrahydro-5,5,8,8-tétraméthyl-2-napht[ 2,3-d ] oxazolyl) benzoïque,
- l'ester méthylique de l'acide p-(5,6,7,8-tétrahydro-5,5,8,8-tétraméthyl-2-napht[ 2,3-d ]imidazolyl) benzoïque,
- l'acide p-(5,6,7,8-tétrahydro-5,5,8,8-tétraméthyl-2-napht [ 2,3-d ]imidazolyl) benzoïque,
- le 2-(4-méthyl) phényl-5,6,7,8-tétrahydro-5,5,8,8-tétraméthylnapht[ 2,3-d ]imidazole.

Parmi les composés de formule (I) particulièrement préférés selon l'invention, on peut mentionner ceux correspondant à la formule générale suivante:

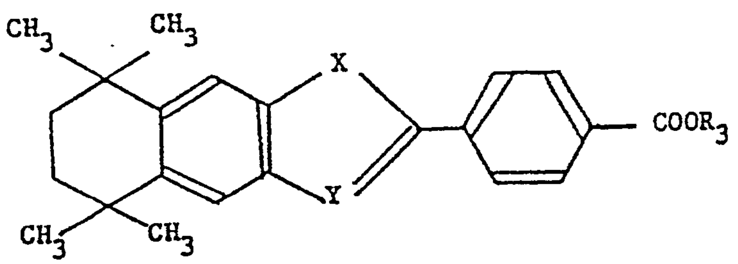

(II)

dans laquelle:

$R_3$ représente un atome d'hydrogène ou un radical alkyle inférieur,

X représente un atome d'oxygène, un atome de soufre ou le radical $NR_4$, et Y représente $CR_4$ ou un atome d'azote, $R_4$ représentant un atome d'hydrogène ou le radical méthyle.

Parmi les composés correspondant à la formule (II) ci-dessus, on peut notamment citer :

- l'acide p-(5,6,7,8-tétrahydro-5,5,8,8-tétraméthyl-2-naphto [ 2,3-b ]thiényl) benzoïque et son ester méthylique,
- l'acide p-(5,6,7,8-tétrahydro-5,5,8,8-tétraméthyl-2-1H-benz [ f ]indolyl) benzoïque et son ester méthylique,
- l'acide p-(5,6,7,8-tétrahydro-5,5,8,8-tétraméthyl-2-napht [ 2,3-d ]oxazolyl) benzoïque et son ester méthylique,
- l'acide p-(5,6,7,8-tétrahydro-5,5,8,8-tétraméthyl-2-napht [ 2,3-d ]imidazolyl benzoïque et son ester méthylique, et
- l'acide p-(1-méthyl 5,6,7,8-tétrahydro-5,5,8,8-tétraméthyl-2-benz[ f ]indolyl) benzoïque et son ester méthylique.

La présente invention a également pour objet le procédé de préparation des composés de formule (I).

Ces composés peuvent être obtenus selon deux voies de synthèse:

A) Première méthode (schéma I).

Cette méthode est tout particulièrement préférée lorsque dans les composés de formule (I) le radical Y représente un atome d'azote.

Cette méthode consiste à faire réagir un dérivé d'acide carboxylique aromatique de formule (1) sur un dérivé aromatique diamino, hydroxyamino ou thioamino de formule (2).

SCHEMA I

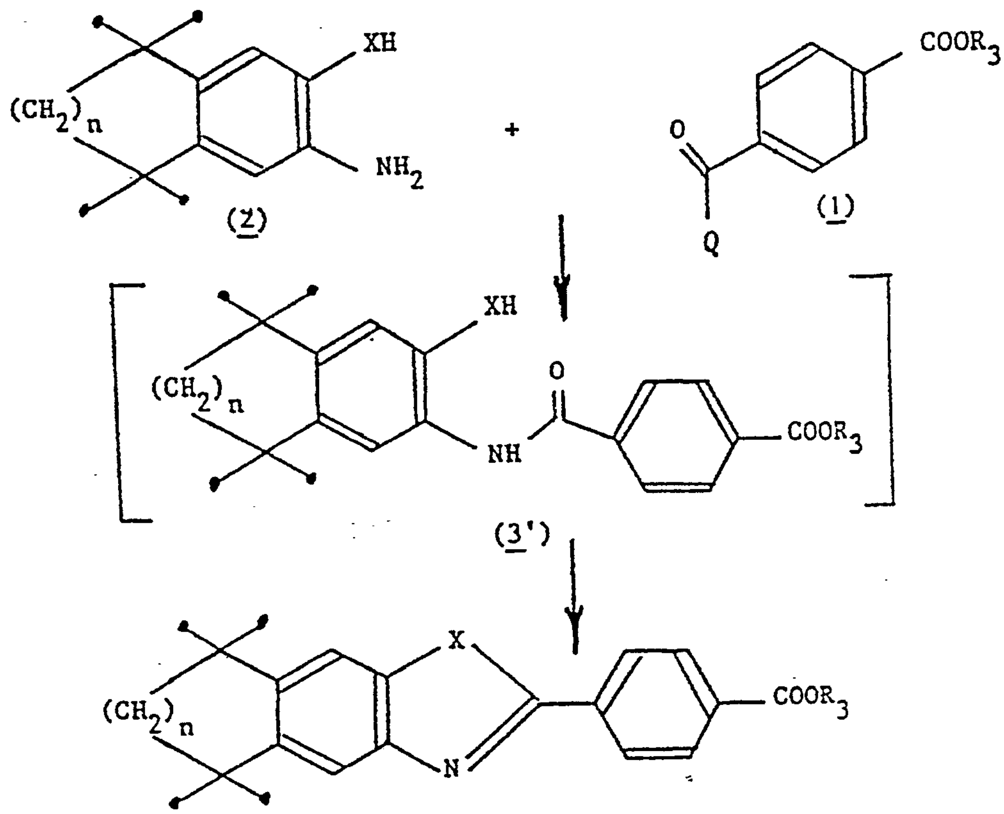

(2) (1) (3') (3)

$R_3 \neq H$

Q= OH ou Cl

X=NH, O ou S

L'action du chlorure d'acide (1) (Q=Cl) sur le composé amino aromatique (2) conduit à l'intermédiaire (3') que l'on isole. De même le couplage de l'acide (1) (Q=OH) avec le composé (2), en présence de triphénylphosphine et de dicyclohexylcarbodiimide, ou de diéthylazodicarboxylate et de triphénylphosphine, de manière connue, conduit aux composés intermédiaires (3').

Ce composé intermédiaire (3') est ensuite cyclisé par traitement acide pour conduire aux composés de formule (3). On peut utiliser pour cette cyclisation un acide sulfonique tel que l'acide p-toluène sulfonique dans un solvant inerte tel que le toluène ou le xylène. La température de cyclisation est de préférence voisine de la température de reflux du solvant utilisé.

Selon une variante de ce procédé, il est possible d'accéder directement aux composés de formule (3) par chauffage direct de l'acide de formule (1) (Q=OH) et du composé amino aromatique (2) dans un solvant inerte tel que le xylène en présence d'un cathalisateur acide par exemple l'acide p–toluène sulfonique et ceci au reflux du solvant.

B/ Deuxième méthode (schéma II)

Cette méthode est tout particulièrement préférée lorsque dans les composés de formule (I) Y représente le radical $CR_4$.

SCHEMA .II

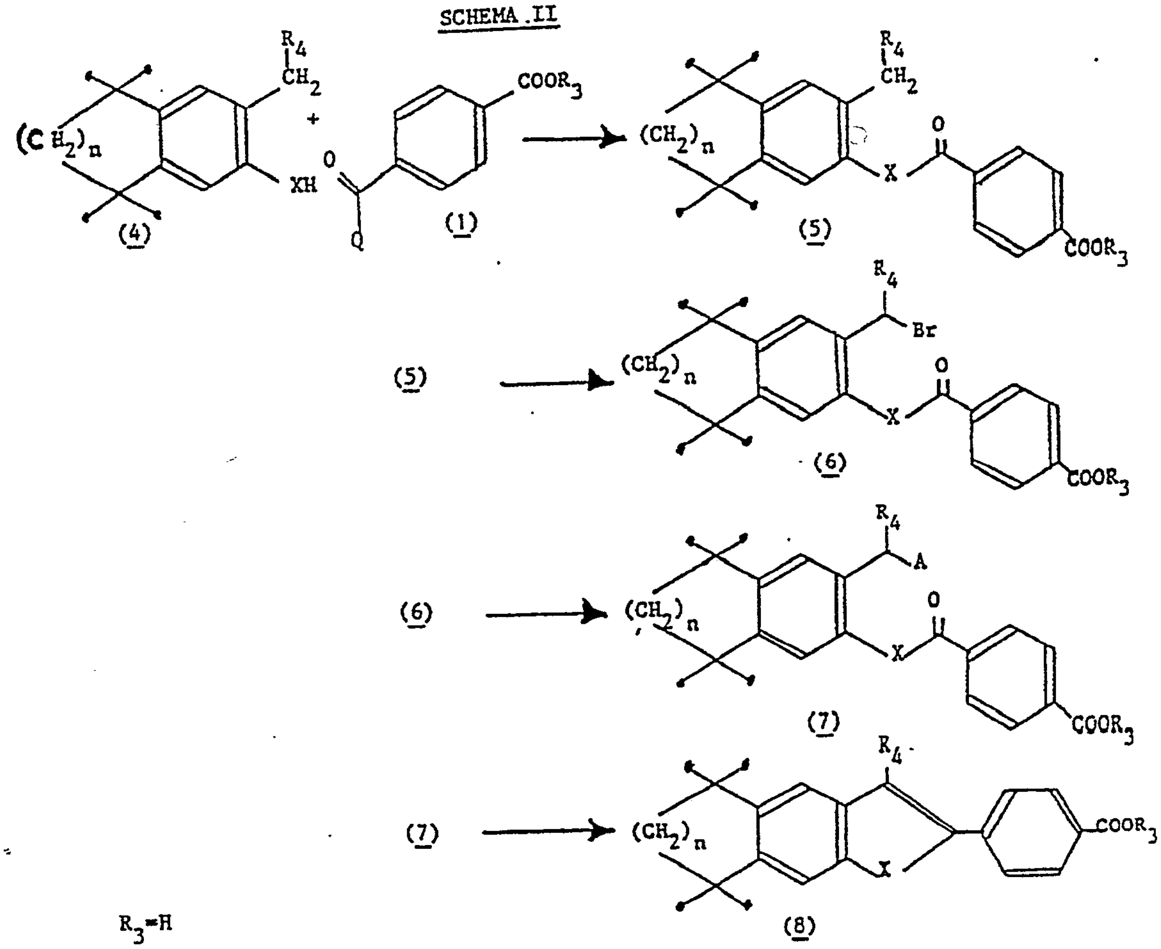

(4) (1) (5) (5) (6) (6) (7) (7) (8)

$R_3$=H

Q=OH ou Cl

X= NH, O ou S

A= $-\overset{+}{P}(V)_3\ Br^-$, V étant un alkyle ou un aryle

ou $-\underset{\underset{O}{\|}}{P}-(W)_2$ , W étant un aryle, un alkoxy ou un aryloxy

Selon cette deuxième méthode, la réaction de cyclisation c'est à dire le passage du dérivé (7) porteur d'un groupement phosphonium ou phosphinyle,au composé (8), est effectuée selon les conditions de la réaction de Wittig ou Wittig-Horner c'est-à-dire en présence d'une base qui peut être un hydroxyde ou un carbonate de métal alcalin par exemple la lithine ou le carbonate de potassium, un hydrure de métal alcalin par exemple l'hydrure de sodium, un alcoolate de métal alcalin par exemple le méthylate de sodium ou le tert-butoxyde de potassium, une amine tertiaire par exemple la triéthylamine, la di-isopropyléthylamine ou le diazabicycloundécène (DBU) ou encore un amidure alcalin par exemple l'amidure de sodium ou le di-isopropylamidure de lithium. La température de la réaction est comprise entre -78°C à +150°C et on peut utiliser comme solvant, un solvant aprotique dipolaire (diméthylsulfoxyde ou diméthylformamide), un alcool, un éther (dioxanne ou tétrahydrofuranne). La réaction est avantageusement effectuée dans le tétrahydrofuranne (THF) entre 0°C et 80°C en utilisant la triéthylamine ou le DBU comme base.

La réaction de bromation c'est-à-dire l'obtention du composé de formule (6) est effectuée en présence de N-bromosuccinimide dans le benzène ou le tétrachlorure de carbone préalablement séché, la température étant de préférence comprise entre 70°C et 90°C, l'initiateur radicalaire étant de préférence le peroxyde de benzoyle.

La réaction d'acylation c'est-à-dire l'obtention du composé de formule (5) est effectuée de manière classique. Lorsque X représente un groupement NH, la réaction est avantageusement effectuée en utilisant le composé de formule (1) sous forme de chlorure d'acide (Q=Cl) en présence d'une amine tertiaire.

Les composés obtenus selon les deux méthodes décrites ci-dessus peuvent être convertis de manière classique en vue d'obtenir l'une quelconque des autres significations de $R_1$.

Ainsi la saponification des esters donne les acides correspondants. Ceux-ci peuvent être transformés en chlorures d'acides qui sont alors facilement convertis en amides. Ces amides peuvent également être obtenus par action directe d'amines sur les esters obtenus précédemment. La réduction des esters, aldéhyde ou amide par un agent réducteur convenable (par exemple l'aluminohydrure de lithium) permet l'accès aux alcools et amines correspondants.

La présente invention a également pour objet à titre de produits industriels nouveaux les intermédiaires de synthèse correspondant à la formule générale suivante:

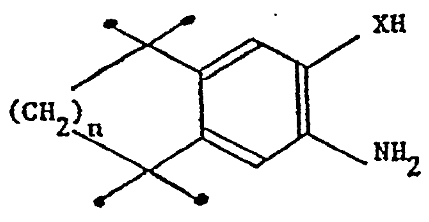

(III)

dans laquelle:

n est 1 ou 2

et X représente un atome d'oxygène, un atome de soufre ou le radical $NR_4$, R4 représentant un radical alkyle inférieur.

Parmi les composés intermédiaires de formule (III) ci-dessus on peut notamment citer :

– le 3-amino-5, 6, 7, 8-tétrahydro-5, 5, 8, 8-tétraméthyl-2-naphtol.

L'état de la technique se rapportant aux intermédiaires de Formule (III) est essentiellement représenté par le Brevet FR 8 115 936 (2 488 890) ainsi que par le Chemical Abstracts, Vol. 52, n° 16, 13694 (f) 1958.

Les composés selon l'invention présentent une bonne à excellente activité dans le test d'inhibition de l'ornithine décarboxylase, chez le rat nu après induction par le "tape stripping ". (Mi. BOUCLIER et coll., DERMATOLOGICA 169 n° 4 1984). Ce test est admis comme mesure de 1, action des rétinoïdes sur les phénomènes de prolifération cellulaire.

Ces composés conviennent particulièrement bien pour traiter les affections dermatologiques liées à un désordre de la kératinisation (différenciation, prolifération) ainsi que les affections dermatologiques à composante inflammatoire et/ou immunoallergique notamment:

– les acnés vulgaires, comédoniennes ou polymorphes, les acnés séniles, solaires, et les acnés médicamenteuses ou professionnelles,
– les kératites solaires
– les formes étendues et/ou sévères de psoriasis, et les autres troubles de la kératinisation, et notamment: les ichtyoses et états ichtyosiformes,
– la maladie de Darier,
– les kératodermies palmo-plantaires,
– les leucoplasies et états leucoplasiformes, le lichen plan,
– toutes proliférations dermatologiques bénignes ou malignes, sévères ou étendues.

Ils sont également actifs pour certaines affections rhumatismales dans le traitement des tumeurs, du psoriasis rhumatoide, des atopies cutanées ou respiratoires ainsi que dans le traitement de certains problèmes ophtalmologiques relatifs aux cornéopathies.

Ces composés peuvent être utilisés également pour lutter contre le vieillissement de la peau en particulier sous l'effet du soleil.

La présente invention a donc également pour objet des compositions médicamenteuses contenant au moins un composé de formule (I) tel que défini ci-dessus ou l'un de ses sels.

La présente invention a donc aussi pour objet une nouvelle composition médicamenteuse, destinée notamment au traitement des affections sus-mentionnées, caractérisée par le fait qu'elle comporte, dans un support pharmaceutique acceptable, au moins un composé de formule (I) ou l'un de ses sels.

Les composés selon l'invention sont généralement administrés à une dose journalière d'environ 0,01µg/Kg à 1mg/Kg de poids corporel.

Comme support des compositions, on peut utiliser tout support conventionnel, le composé actif se trouvant soit à l'état dissous soit à l'état dispersé dans le véhicule.

L'administration peut être effectuée par voie entérale, parentérale, rectale, topique ou oculaire. Par voie entérale, les médicaments peuvent se présenter sous forme de comprimés, de gélules, de dragées, de sirops, de suspensions, de solutions, de poudres, de granulés, d'émulsions. Par voie parentérale, les compositions peuvent se présenter sous forme de solutions ou suspensions pour perfusion ou pour injection. Par voie rectale, les compositions se présentent sous forme de suppositoires. Par voie topique, les compositions pharmaceutiques à base des composés selon l'invention, se présentent sous forme d'onguents, de teintures, de crèmes, de pommades, de poudres, de timbres, de tampons imbibés, de solutions, de lotions, de gels, de sprays ou encore de suspensions. Les compositions par voie topique contiennent de préférence de 0,00001 à environ 0,01% en poids de composé(s) de formule (I). Ces compositions par voie topique peuvent se présenter soit sous forme anhydre soit sous forme aqueuse selon l'indication clinique. Par voie oculaire, ce sont principalement des collyres.

Les composés de formule (I), selon l'invention, trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les effets néfastes du soleil ou dans le traitement des peaux physiologiquement sèches.

La présente invention vise donc également une composition cosmétique contenant, dans un support cosmétiquement acceptable, au moins un composé de formule (I) ou un de ses sels, cette composition se présentant notamment sous forme de lotion, gel, savon ou shampooing.

La concentration en composé de formule (I), dans les compositions cosmétiques est comprise entre 0,00001 et 0,01 % en poids.

Les compositions médicamenteuses et cosmétiques selon l'invention, peuvent contenir des additifs inertes ou même pharmacodynamiquement ou cosmétiquement actifs et notamment: des agents hydratants comme la thiamorpholinone et ses dérivés ou l'urée; des agents antiséborrhéiques ou antiacnéiques tels que la S-carboxyméthylcystéine, la S-benzylcystéamine, leurs sels et leurs dérivés,la tioxolone ou le peroxyde de benzoyle; des antibiotiques comme l'érythromycine et ses esters, la néomycine, les tétracyclines ou les polyméthylène-4-isothiazoline-4 ones-3 ;des agents favorisant la repousse des cheveux, comme le "Minoxidil" (diamino-2,4 pipéridino-6 pyrimidine oxyde-3) et ses dérivés, le Diazoxide (chloro-7 méthyl-3 benzothiadiazine-1,2,4 dioxyde-1,1) et le Phénytoïn (5,5-diphénylimidazolidine-2,4 dione) ; des agents anti-inflammatoires stéroïdiens et non stéroïdiens; des caroténoïdes et notamment le β-carotène; des agents anti-psoriasiques tels que l'anthraline et ses dérivés, les acides eicosatétraynoïque 5,8,11,14 et eicosatriynoïque-5,8,11, leurs esters et amides.

Les compositions selon l'invention peuvent également contenir des agents d'amélioration de la saveur, des agents conservateurs, des agents stabilisants, des agents régulateurs d'humidité, des agents régulateurs de pH, des agents modificateurs de pression osmotique, des agents émulsionnants, des anti-oxydants tels que l'α -tocophérol, le butylhydroxy-anisole ou le butylhydroxytoluène.

On donne ci-après à titre d'illustration et sans aucun caractère limitatif, plusieurs exemples de préparation des composés actifs de formule (I) selon l'invention ainsi que des exemples de compositions les contenant.

## EXEMPLE 1

Ester méthylique de l'acide p-(5,6,7,8-tétrahydro-,5,5,8,8-tétraméthyl-2 -naphto 2,3-b furanyl) benzoïque

a) 3-Méthyl-5,6,7,8-tétrahydro-5,5,8,8-tétraméthyl-2-naphtol.

Dans un ballon, on introduit 10,8g (100mmoles) d'ortho crésol, 100ml de dichlorométhane ($CH_2Cl_2$) et 18,3g (100mmoles) de 2,4-dichloro-2,4-diméthylhexane. On refroidit à 5°C et ajoute par petites quantités 6,6g (50mmoles) de chlorure d'aluminium. On laisse remonter à 20°C. On agite 2h puis verse le milieu réactionnel dans 200ml d'eau. On décante la phase organique, sèche sur sulfate de magnésium (Mg $SO_4$), et évapore les solvants. Le résidu est recristallisé dans 100ml d'hexane. On obtient ainsi 20,3g (93%) de 3-méthyl-5,6,7,8-tétrahydro-5,5,8,8-tétraméthyl-2-naphtol qui fond à 122-123°C.

b) Ester méthylique de l'acide p-(3-méthyl-5,6,7,8-tétrahydro-5,5,8,8-tétraméthyl-2-naphtyloxycarbonyl) benzoïque.

Dans 100ml de tétrahydrofuranne (THF), on dissout 8,7g (40mmoles) de naphtol obtenu en 1 a) et 6,2ml (44mmoles) de triéthylamine. On ajoute goutte à goutte une solution de chlorure de p-(méthoxycarbonyl) benzoyle (8,8g = 44mmoles) dans du THF (50ml), et agite 4h à température ambiante. On verse le milieu réactionnel dans 200ml d'eau et extrait avec 300ml de $CH_2Cl_2$). On décante la phase organique, sèche sur Mg $SO_4$, et évapore les solvants. Le résidu est recristallisé dans l'isooctane. On obtient ainsi 11g (72%) d'ester méthylique de l'acide p-(3-méthyl-5,6,7,8-tétrahydro-5,5,8,8-tétraméthyl-2-naphtyloxy carbonyl) benzoïque, qui fond à 111-112°C.

c) Ester méthylique de l'acide p-(3-bromométhyl-5,6,7,8-tétrahydro-5,5,8,8-tétraméthyl-2-naphtyloxy carbonyl) benzoïque.

Un mélange de l'ester obtenu en 1 b) (10,60g; 27,8mmoles), de peroxyde de benzoyle (50mg) et de tétrachlorure de carbone ($CCl_4$), (150ml) est porté au reflux. On ajoute par petites quantités 4,96g (27,8mmoles), de N-bromo succinimide (NBS).

On maintient le reflux pendant 24h et évapore le solvant. Le résidu est purifié par passage sur une colonne de silice (éluant: mélange hexane/$CH_2Cl_2$ : 1/1). On recueille ainsi 12g d'un mélange contenant 80% de l'ester attendu et 20% de l'ester de départ.

d) Ester méthylique de l'acide p-(5,6,7,8-tétrahydro-5,5,8,8-tétraméthyl-naphto[ 2,3-b ]furan-2-yl) benzoïque.

Dans un ballon, on introduit 11,8g du mélange obtenu en 1 c), 100ml de THF, et 6,50g (24,6mmoles) de triphénylphosphine. On chauffe à reflux durant 4h, refroidit à 10°C, et ajoute goutte à goutte 3,70ml (24,6mmoles) de 1,8-diazabicyclo (5.4.0) undec-7-ène (DBU). On laisse revenir à température ambiante et agite pendant 5h. On verse le milieu réactionnel dans l'eau, extrait à l'éther, sèche sur Mg $SO_4$ et évapore les solvants.

Le résidu est purifié par passage sur colonne de silice (éluant: hexane/$CH_2Cl_2$ : 1/1). On obtient ainsi 4,20g d'ester méthylique de l'acide p-(5,6,7,8-tétrahydro-5,5,8,8-tétraméthyl-2-naphto[ 2,3-b ]furanyl) benzoïque qui fond à 184-185°C.

EXEMPLE 2

Acide p-(5,6,7,8-tétrahydro-5,5,8,8-tétraméthyl-2-naphto [ 2,3-b ]furanyl) benzoïque.

3,80g (10,4mmoles) de l'ester obtenu en 1 d) sont traités au reflux pendant 4h par 200ml de soude méthanolique 2N. On évapore le méthanol, reprend par l'eau et acidifie avec de l'acide chlorhydrique (HCl) concentré. On extrait à l'éther, décante la phase organique, sèche sur Mg $SO_4$, évapore les solvants. Le résidu est recristallisé dans un mélange d'éther diisopropylique et d'acétate d'éthyle 2/1. On obtient ainsi 3,50g (97%) d'acide p-(5,6,7,8-tétrahydro-5,5,8,8-tétraméthyl-2-naphto[ 2,3-b ]furanyl) benzoïque qui fond à 307-312°C.

EXEMPLE 3

Ester méthylique de l'acide p-(5,6,7,8-tétrahydro-5,5,8,8-tétraméthyl-2-naphto[ 2,3-b ]thiényl) benzoïque.

a) 2-(N,N-diméthylthiocarbamoyloxy)-3-méthyl-5,6,7,8-tétrahydro-5,5,8,8 tétraméthyl-naphtalène.

Dans un ballon, on introduit 1,7g (57mmoles) d'hydrure de sodium (80% dans l'huile) et 50ml de diméthylformamide. On ajoute goutte à goutte une solution de 10,3g (47mmoles) de 3-méthyl-5,6,7,8-tétrahydro-5,5,8,8-tétraméthyl-2-naphtol dans 100ml de DMF et agite jusqu'à cessation du dégagement gazeux. On ajoute 8,1g (66mmoles) de chlorure de diméthylthio-carbamoyle dans 100ml de DMF et agite 4h à température ambiante. On verse dans l'eau, extrait à l'éther éthylique, décante la phase organique, lave à l'eau, sèche sur Mg $SO_4$, et évapore. Le résidu est purifié par chromatographie sur colonne de silice (éluant: $CH_2Cl_2$/hexane: 20/80). On obtient ainsi 13,2g (92%) de 2-(N,N-diméthylthiocarbamoyloxy)-3-méthyl-5,6,7,8-tétrahydro-5,5,8,8-tétraméthyl-naphtalène, qui fond à 102-103°C.

b) 2-(N,N-diméthylcarbamoylthio)-3-méthyl-5,6,7,8-tétrahydro-5,5,8,8-tétraméthyl-naphtalène.

13g (42,5mmoles) du composé obtenu en 3 a) sont chauffés sous azote à 280°C. Après refroidissement, le résidu est passé sur colonne de silice (éluant: hexane/$CH_2Cl_2$ 70/30).

On obtient ainsi 10,2g (79%) de 2-(N,N-diméthylcarbamoylthio)-3-méthyl-5,6,7,8-tétrahydro-5,5,8,8-tétraméthyl-naphtalène qui fond à 142-143°C.

c) 3-méthyl-5,6,7,8-tétrahydro-5,5,8,8-tétraméthyl-2-thionaphtol.

9,2g (30mmoles) du produit obtenu en 3 b) sont traités au reflux par 200ml de soude méthanolique 2N, pendant 2h. On évapore les solvants, reprend par l'eau, acidifie à pH 0 (HCl concentré), extrait à l'éther, décante la phase organique, sèche sur sulfate de magnésium et évapore les solvants. On obtient ainsi 6,9g (98%) de 3-méthyl-5,6,7,8-tétrahydro-5,5,8,8-tétraméthyl-2-thionaphtol, qui fond à 91-92°C.

d) Ester méthylique de l'acide p- (3-méthyl-5,6,7,8-tétrahydro-5,5,8,8-tétraméthyl-2-naphtylthio) carbonyl benzoïque.

Dans un ballon, on introduit 5,9g (33mmoles) de l'ester monométhylique de l'acide téréphtalique et 50ml de THF. On ajoute par petites portions 5,3g (33mmoles) de 1,1'-carbonyldiimidazole et agite jusqu'à cessation du dégagement gazeux. On ajoute ensuite 7g (30mmoles) du composé obtenu en 3 c) dans 50ml de THF et agite pendant 4h à température ambiante.

On verse dans l'eau, extrait à l'éther, lave avec une solution saturée de bicarbonate de sodium, décante la phase organique, sèche sur Mg $SO_4$, et évapore. Le résidu est recristallisé dans l'isooctane

pour donner 9,5g (81%) d'ester méthylique de l'acide p- (3-méthyl-5,6,7,8-tétrahydro-5,5,8,8-tétraméthyl-2-naphtylthio) carbonyl benzoïque qui fond à 105-106°C.

e) Ester méthylique de l'acide p- (3-bromométhyl-5,6,7,8-tétrahydro-5,5,8,8-tétraméthyl-2-naphtylthio) carbonyl benzoïque.

9,26g (23mmoles) du composé préparé en 3 d) sont chauffés à reflux dans 150ml de tétrachlorure de carbone contenant 50mg de peroxyde de benzoyle. On ajoute par petites quantités 4,16g (23mmoles) de N-bromo-succinimide et, une fois l'addition terminée, on maintient le reflux pendant 12h. On évapore le solvant et purifie le résidu par chromatographie sur colonne de silice (éluant: $CH_2Cl_2$/hexane 1/1). On obtient ainsi 10,8g d'un mélange du dérivé monobromé attendu (85%) et des dérivés non bromé et dibromé (15%). (Dosage effectué en comparant les intégrations de signaux en RMN du proton des groupes méthyle, bromométhyle et dibromométhyle dans les composants du mélange). Ce mélange est utilisé tel quel pour la suite de la synthèse.

f) Ester méthylique de l'acide p-(5,6,7,8-tétrahydro-5,5,8,8-tétraméthyl-2-naphto[ 2,3-b ]thiényl) benzoïque.

Dans un ballon, on introduit 10,5g du mélange précédent, 6g (23mmoles) de triphénylphosphine, et 100ml de THF. On chauffe à reflux durant 4h, refroidit à 10°C, et rajoute 3,5ml (23mmoles) de DBU. On agite 4h à température ambiante, verse dans l'eau, extrait à l'éther, décante la phase organique, sèche sur Mg $SO_4$ et évapore. Le résidu est purifié par chromatographie sur colonne de silice (éluant: hexane/dichlorométhane 80/20). On obtient ainsi 6,6g d'ester méthylique de l'acide p-(5,6,7,8-tétrahydro-5,5,8,8-tétraméthyl-2-naphto[ 2,3,-b ]thiényl) benzoïque qui fond à 186-187°C.

EXEMPLE 4

Acide p-(5,6,7,8-tétrahydro-5,5,8,8-tétraméthyl-2-naphto [2,3,-b]thiényl) benzoïque.

5g (13mmoles) de l'ester obtenu en 3 f) sont traités par 200ml de soude méthanolique 2N. On chauffe à reflux 2h, évapore à sec, reprend par l'eau, acidifie à pH=1 avec HCl concentré, extrait à l'éther, décante la phase organique, sèche sur Mg $SO_4$, et évapore. Le résidu est recristallisé dans un mélange éther isopropylique-acétate d'éthyle (2/1). On obtient ainsi 4,6g (96%) d'acide p-(5,6,7,8-tétrahydro-5,5,8,8-tétraméthyl-2-naphto [ 2,3,-b ]thiényl) benzoïque qui fond à 291-292°C.

EXEMPLE 5

Acide p-(5,6,7,8-tétrahydro-5,5,8,8-tétraméthyl-2-1H-benz [ f ]indolyl) benzoïque.

a) 2-méthyl-5,6,7,8-tétrahydro-5,5,8,8-tétraméthyl-naphtalène.

Dans un ballon, on introduit 64ml (600mmoles) de toluène et 36,6g (200mmoles) de 2,5-dichloro-2,5-diméthyl-hexane. On refroidit à 0°C et ajoute par petites quantités 4,1g (30mmoles) de chlorure d'aluminium, agite 1h à température ambiante, verse le milieu réactionnel dans l'eau, extrait au $CH_2Cl_2$, décante la phase organique, sèche sur sulfate de magnésium et évapore. L'huile obtenue est purifiée par distillation. On obtient ainsi 39,4g (98%) de 2-méthyl-5,6,7,8-tétrahydro-5,5,8,8-tétraméthyl-naphtalène qui bout à 68°C (sous 1mm de mercure).

b) 2-méthyl-3-nitro-5,6,7,8-tétrahydro-5,5,8,8-tétraméthyl-naphtalène.

50g (250mmoles) de 2-méthyl-5,6,7,8-tétrahydro-5,5,8,8-tétraméthyl-naphtalène sont dissous dans 200ml d'anhydride acétique. On refroidit à 0°C et ajoute goutte à goutte une solution de 10,5ml (250mmoles) d'acide nitrique, 20ml d'acide acétique et 20ml d'anhydride acétique, en maintenant la température entre 0 et 5°C. On agite ensuite 1h à température ambiante, verse dans l'eau glacée, filtre le solide obtenu et lave à l'eau. On dissout le solide dans du chlorure de méthylène, lave à l'eau, puis avec une solution saturée de bicarbonate de sodium, sèche sur Mg $SO_4$ et évapore. On obtient ainsi 45,8g (74%) de 2-méthyl-3-nitro-5,6,7,8-tétrahydro-5,5,8,8-tétraméthyl-naphtalène qui fond à 143-144°C.

c) 3-méthyl-5,6,7,8-tétrahydro-5,5,8,8-tétraméthyl-2-naphtylamine.

24,7g (100mmoles) du dérivé nitré obtenu en 5 b) sont dissous dans 400ml d'éthanol. On ajoute 33,6g (600mmoles) de fer en poudre, puis, goutte à goutte, 38ml d'acide chlorhydrique concentré. On chauffe à

reflux pendant 1h, évapore à sec, reprend le résidu par l'eau, ajoute avec précaution un excès de bicarbonate de sodium, puis extrait à l'éther. On filtre, récupère le filtrat, sèche sur Mg $SO_4$, et évapore. On obtient ainsi 21,2g (98%) de 3-méthyl-5,6,7,8-tétrahydro-5,5,8,8-tétraméthyl-2-naphtylamine, qui fond à 94-95°C.

d) Ester méthylique de l'acide p-[(3-méthyl-5,6,7,8-tétrahydro-5,5,8,8 tétraméthyl-2-naphtyl)carbamoyl] benzoïque.

8,1g (45mmoles) de monotéréphtalate de méthyle sont dissous dans 100ml de THF. On ajoute par petites quantités 8g (45mmoles) de 1,1'carbonyldiimidazole. On agite jusqu'à ce que le dégagement gazeux ait cessé, puis, on ajoute goutte à goutte une solution de 9,8g (45mmoles) de l'amine obtenue en 5 c) dans 50ml de THF. On agite 2h à température ambiante, verse dans l'eau, extrait avec $CH_2Cl_2$, décante la phase organique, sèche sur Mg $SO_4$ , et évapore.

Le résidu est recristallisé dans l'éther isopropylique pour donner 14,6g (86%) de l'ester méthylique de l'acide p-[ (3-méthyl-5,6,7,8-tétrahydro-5,5,8,8-tétraméthyl-2-naphtyl)carbamoyl] benzoïque, qui fond à 169-170°C.

e) Ester méthylique de l'acide p-[ (N-tert-butoxycarbonyl-3-méthyl-5,6,7,8-tétrahydro-5,5,8,8-tétraméthyl-2-naphtylcarbamoyl)]benzoïque.

Dans un ballon, on introduit 1,2g (40,5mmoles) d'hydrure de sodium (80% dans l'huile), 20ml de DMF, et 30ml de THF. On ajoute goutte à goutte une solution de 13,9g (37mmoles) de l'ester obtenu en 5 d) dans 60ml de THF, et agite jusqu'à ce que le dégagement gazeux ait cessé. On ajoute ensuite 8,8g (40,5mmoles) de di-tert-butyl-dicarbonate dans 100ml de THF et agite à température ambiante pendant 4h. On verse dans l'eau, extrait à l'éther éthylique, décante la phase organique, lave à l'eau, sèche sur Mg $SO_4$ , et évapore. Le résidu est purifié par chromatographie sur colonne de silice (éluant: mélange dichlorométhane-hexane 7/3). On obtient ainsi 14,1g (82%) de l'ester méthylique de l'acide p-[(N-tert-butoxycarbonyl-3-méthyl-5,6,7,8-tétrahydro-5,5,8,8-tétraméthyl-2-naphtylcarbamoyl)]benzoïque, qui fond à 176-177°C.

f) Ester méthylique de l'acide p-(3-bromométhyl-N-tert-butoxycarbonyl-5,6,7,8-tétrahydro-5,5,8,8-tétraméthyl-2-naphtylcarbamoyl) benzoïque.

13,25g (27,6mmoles) de l'ester obtenu en 5 e) sont placés dans un ballon. On ajoute 150ml de tétrachlorure de carbone et 50mg de peroxyde de benzoyle. On chauffe à reflux et introduit par petites quantités, 4,9g (27,6mmoles) de N-bromosuccinimide. Le reflux est maintenu pendant 12h. On évapore le solvant et purifie le résidu par chromatographie sur silice, en éluant avec un mélange de dichlorométhane et d'hexane : 1/1. On obtient ainsi 14,2g d'un mélange contenant environ 85% du dérivé monbromé attendu et 15% d'un mélange de produit dibromé et de produit de départ (ces proportions sont estimées par RMN, de manière analogue à celle de l'exemple 3 e). Le mélange est utilisé tel quel pour la suite de la synthèse.

g) Ester méthylique de l'acide p-(N-tert-butoxycarbonyl-5,6,7,8-tétrahydro-5,5,8,8-tétraméthyl-2-IH-benz [f] indolyl) benzoïque.

Dans un ballon, on introduit 13,9 g du mélange obtenu en 5 f), 6,7 g (25,5 mmolas) de triphényl phosphine, et 100 ml de THF. On chauffe à reflux durant 8 h, refroidit à 5°C, et ajoute goutte à goutte 3,8 ml (25,5 mmoles) de DBU. On agite à température ambiante pendant 2 h, verse le milieu réactionnel dans l'eau, extrait à l'éther, décante la phase organique, sèche sur Mg $SO_4$ et évapore. Le résidu est purifié par chromatographie sur colonne de silice (éluant: mélange $CH_2$ $Cl_2$-hexane 20.80). On obtient ainsi 6,7 g (69%) d'ester méthylique de l'acide p-(N-tert-butoxycarbonyl-5,6,7,8-tétrahydro-5,5,8,8-tétraméthyl-2-IH-benz [f] indolyl) benzoïque, qui fond à 145-146°C.

h) Acide p-(5,6,7,8-tétrahydro-5,5,8,8-tétraméthyl-2-IH-benz [f] indolyl) benzoïque.

6,4 g (14 mmoles) de l'ester obtenu en 5 g) sont traités au reflux pendant 4 h par 100 ml de soude méthanolique 2N. On évapore le solvant, reprend par l'eau, acidifie la phase aqueuse jusqu'à pH 5 avec de l'acide chlorhydrique, extrait à l'éther éthylique, décante la phase organique, sèche sur Mg $SO_4$, et évapore. Le résidu est trituré dans 100 ml d'hexane. On obtient ainsi 4,3 g (90%) d'acide p-(5,6,7,8-tétrahzdro-5,5,8,8-tétraméthyl-2-IH-benz [f] indolyl) benzoïque, qui fond à 294-296°C.

<u>EXAMPLE 6</u>

<u>Ester méthylique de l'acide p-(5,6,7,8-tétrahydro-5,5,8,8-tétraméthyl-2-IH-benz [f] indolyl) benzoïque.</u>

.2,3 g (6,6 mmoles) d'acide p-(5,6,7,8-tétrahydro-5,5,8,8-tétraméthyl-2-IH-benz [f] indolyl) benzoïque sont placés dans un ballon contenant 150ml de méthanol. On ajouta goutte à goutte 2 ml d'acide sulfurique concentré et chauffs à reflux pendant 4 h. On évapore à sec, reprend par l'eau et alcalinise au bicarbonate de sodium. On extrait au chlorure de méthylène, décante la phase organique, sèche sur Mg $SO_4$, et évapore. On obtient ainsi 2,3 g (96%) d'ester méthylique de l'acide p-(5,6,7,8-tétrahydro-5,5,8,8-tétraméthyl-2-IH-benz [f] indolyl) benzoïque, qui fond à 212-213°C.

EXEMPLE 7

Ester méthylique de l'acide p-(1-méthyl-(5,6,7,8-tétrahydro-5,5,8,8-tétraméthyl-2-benz[ f ]indolyl) benzoïque.

180mg (5,1mmoles) d'hydrure de sodium (80% dans l'huile), sont mis en suspension dans 20ml de DMF. On ajoute goutte à goutte 1,8g (5mmoles) de l'ester préparé à l'exemple 6, dissous dans 5ml de THF et agite jusqu'à ce que le dégagement gazeux ait cessé. On ajoute ensuite 0,4ml (6,4mmoles) d'iodure de méthyle et agite 2h à température ambiante. On verse dans l'eau, extrait au $CH_2Cl_2$, décante la phase organique, sèche sur Mg $SO_4$ et évapore. Le résidu est purifié par chromatographie sur colonne de silice en éluant avec un mélange de dichlorométhane et d'hexane (4/1). Après évaporation des solvants, on obtient 1,4g (78%) d'ester méthylique de l'acide p-(1-méthyl 5,6,7,8-tétrahydro-5,5,8,8-tétraméthyl-2-benz [ f ]indolyl) benzoïque, qui fond à 147-148°C.

EXEMPLE 8

Acide p-(1-méthyl 5,6,7,8-tétrahydro-5,5,8,8-tétraméthyl-2-benz[ f ]indolyl) benzoïque.

1,2g (3,2mmoles) de l'ester obtenu à l'exemple 7 sont traités au reflux pendant 2h par 100ml de soude méthanolique 2N. On chauffe 2h à reflux, évapore à sec, reprend par l'eau, acidifie la phase aqueuse à pH 5 avec de l'acide chlorhydrique, extrait à l'éther, décante la phase organique, sèche sur Mg $SO_4$ et évapore. Le résidu est trituré dans 100ml d'hexane, puis filtré pour donner 1,13g (97%) d'acide p-(1-méthyl 5,6,7,8-tétrahydro-5,5,8,8-tétraméthyl-2-benz[ f ]indolyl) benzoïque, qui fond à 288-289°C.

EXEMPLE 9

Ester méthylique de l'acide p-(5,6,7,8-tétrahydro-5,5,8,8-tétraméthyl-2-naphtl[ 2,3-d]oxazolyl) benzoïque.

a) 5,6,7,8-tétrahydro-5,5,8,8-tétraméthyl-2-naphtol.

54,9g (300mmoles) de 2,5-dichloro-2,5-diméthyl hexane sont dissous dans du dichlorométhane (500ml). On ajoute du phénol (28,2g : 300mmoles) puis du chlorure d'aluminium (8,0g : 60mmoles). On agite énergiquement pendant 16h. On ajoute de l'eau (200ml), et extrait par du dichlorométhane (3x200ml). La phase organique est ensuite lavée avec une solution saturée de bicarbonate de sodium, puis une solution saturée de chlorure de sodium. On sèche sur sulfate de magnésium, filtre, évapore les solvants.

Le solide obtenu est lavé avec un mélange composé d'hexane (80%) et de dichlorométhane (20%) (300ml). On obtient ainsi le 5,6,7,8-tétrahydro-5,5,8,8-tétraméthyl-2-naphtol.: 43,5g (71%), qui fond à 142°C.

b) 3-nitro-5,6,7,8-tétrahydro-5,5,8,8-tétraméthyl-2-naphtol.

Le 5,6,7,8-tétrahydro-5,5,8,8-tétraméthyl-2-naphtol (42,86g; 210mmoles), est solubilisé dans de l'anhydride acétique (420ml).

On refroidit à -10°C, et ajoute de l'acide acétique (40ml). On ajoute de l'acide nitrique fumant (8,7ml; 210mmoles) en solution dans un mélange d'anhydride acétique (40ml) et d'acide acétique (40ml). On agite une heure à température ambiante.

Le milieu réactionnel est versé dans de l'eau (1 litre) + de la glace. Le précipité formé est filtré, lavé à l'eau, repris par du dichlorométhane (3x200ml); la phase organique est lavée avec une solution saturée de bicarbonate de sodium, puis à l'eau. Elle est séchée sur sulfate de magnésium, filtrée, et le solvant évaporé.

Le solide obtenu est purifié par chromatographie sur silice, élué par un mélange de dichlorométhane (50%) et d'hexane (50%).

On obtient ainsi 19,6g (37%) de 3-nitro-5,6,7,8-tétrahydro-5,5,8,8 tétraméthyl-2-naphtol qui fond à 139°C.

c) 3-amino-5,6,7,8-tétrahydro-5,5,8,8-tétraméthyl-2-naphtol.

Le 3-nitro-5,6,7,8-tétrahydro-5,5,8,8-tétraméthyl-2-naphtol: 35,3g; (14mmoles) est mélangé avec du méthanol (1 litre).
On ajoute environ deux spatules de Nickel de Raney lavé au méthanol, et hydrogène jusqu'à fin d'absorption.
Le précipité formé, est solubilisé par addition de dichlorométhane (1 litre).
Le catalyseur est filtré et les solvants évaporés.
Les cristaux obtenus sont lavés par de l'hexane (2 litres), puis filtrés. On obtient ainsi 30,9g (99%) de 3-amino-5,6,7,8-tétrahydro-5,5,8,8 tétraméthyl-2-naphtol qui fond à 225°C.

d) Ester méthylique de l'acide p-[ (3-hydroxy-5,6,7,8-tétrahydro-5,5,8,8 tétraméthyl-2-naphtyl) carbamoyl] benzoïque.

17,5g (80mmoles) de 3-amino-5,6,7,8-tétrahydro-5,5,8,8-tétraméthyl-2-naphtol sont mélangés avec de l'éther éthylique (400ml). On ajoute de la triéthylamine (11,1ml; 80mmoles) puis, goutte à goutte, 15,81g; (80mmoles) de chlorure de p-méthoxy carbonyl benzoyle en solution dans de l'éther (200ml).
On agite 2h à température ambiante, puis verse le milieu réactionnel dans un mélange d'eau (500ml) et de dichlorométhane (300ml).
On extrait la phase aqueuse par du dichlorométhane (2 fois 300ml) et lave la phase organique avec une solution saturée de chlorure de sodium, puis on sèche sur sulfate de magnésium, filtre et évapore le solvant.
Le solide obtenu est purifié par chromatographie sur colonne, élué par du dichlorométhane.
On obtient ainsi: 24,38g (80%) de l'ester méthylique de l'acide p-[ (3-hydroxy-5,6,7,8-tétrahydro-5,5,8,8-tétraméthyl-2-naphtyl)carbamoyl] benzoïque, qui fond à 200-210°C.

e) Ester méthylique de l'acide p-(5,6,7,8-tétrahydro-5,5,8,8-tétraméthyl-2-napht[ 2,3-d ]oxazolyl) benzoïque.

L'ester méthylique de l'acide p-[ (3-hydroxy-5,6,7,8-tétrahydro-5,5,8,8-tétraméthyl-2-naphtyl)carbamoyl] benzoïque : 23,25g (61mmoles), est mélangé avec du xylène (600ml). On ajoute 11,6g (61mmoles) d'acide p.toluène sulfonique et chauffe à reflux, sous agitation pendant 3h. Le xylène est évaporé, et on ajoute de l'eau (500ml) et du dichlorométhane. On ajoute à la phase aqueuse environ 300ml d'une solution saturée de bicar-bonate de sodium et on l'extrait par du dichlorométhane (3 fois 300ml). La phase organique est ensuite lavée avec une solution saturée de bicarbonate de sodium, puis à l'eau.
La phase organique est séchée sur sulfate de magnésium, filtrée et les solvants évaporés.
On purifie le solide obtenu par chromatographie sur colonne, éluée par un mélange de 80% de dichlorométhane et 20% d'hexane.
On obtient ainsi l'ester méthylique de l'acide p-(5,6,7,8-tétrahydro-5,5,8,8-tétraméthyl-2-napht [ 2,3-d ]oxazolyl) benzoïque : 12,25g (54%), qui fond à 174°C.

EXEMPLE 10

Acide p-(5,6,7,8-tétrahydro-5,5,8,8-tétraméthyl-2-napht[ 2,3-d ]oxazolyl) benzoïque.

L'ester méthylique de l'acide p-(5,6,7,8-tétrahydro-5,5,8,8-tétraméthyl-2-napht[ 2,3-d ]oxazolyl) benzoïque (7,27g; 20mmoles) est mélangé à du méthanol (400ml). On ajoute de la soude 5N (40ml), et chauffe à reflux pendant 1h.
Le méthanol est évaporé, et on ajoute de l'éther éthylique (300ml) et de l'acide chlorhydrique 4N (200ml). La phase aqueuse est extraite par de l'éther (2x300ml), et la phase organique est lavée deux fois par de l'eau et une fois par une solution saturée de chlorure de sodium.
La phase organique est séchée sur sulfate de magnésium, filtrée, et le solvant évaporé.
Le solide obtenu est repris avec de l'hexane (300ml) filtré et séché. On obtient ainsi: 7,00g (100%) d'acide p-(5,6,7,8-tétrahydro-5,5,8,8-tétraméthyl-2-naphto[ 2,3-d ]oxazolyl) benzoïque, qui fond à 290°C.

EXEMPLE 11

Alcool p-(5,6,7,8-tétrahydro-5,5,8,8-tétraméthyl-2-napht[ 2,3-d]oxazolyl) benzylique.

L'ester méthylique de l'acide p-(5,6,7,8-tétrahydro-5,5,8,8-tétraméthyl-2-napht[ 2,3-d ]oxazolyl) benzoïque (2,64g ; 7,26mmoles) est solubilisé dans du tétrahydrofuranne sec (50ml), puis additionné goutte

à goutte sur de l'hydrure de lithium et d'aluminium (474mg; 11,9mmoles) en suspension dans du tétrahydrofuranne sec (50ml).

Le milieu réactionnel est chauffé à reflux pendant 5h, refroidi à 0°C, puis hydrolysé par addition goutte à goutte de 30ml d'une solution de tartrate double de sodium et de pottassium.

Le solvant est évaporé, et on ajoute 300ml d'eau et extrait le produit par de l'éther (6x200ml). La phase organique est lavée avec une solution saturée de chlorure de sodium puis séchée sur sulfate de magnésium. Après évaporation du solvant le produit est recristallisé dans l'acétonitrile. On obtient ainsi : 2,16g (89%) d'alcool p-(5,6,7,8-tétrahydro-5,5,8,8-tétraméthyl-2-napht[ 2,3-d ]oxazolyl) benzylique, qui fond à 200°C.

EXEMPLE 12

Aldéhyde p-(5,6,7,8-tétrahydro-5,5,8,8-tétraméthyl-2-napht[ 2,3-d ]oxazolyl) benzoïque.

L'alcool p-(5,6,7,8-tétrahydro-5,5,8,8-tétraméthyl-2-napht [ 2,3-d ]oxazolyl) benzylique (1,24g; 3,7mmoles) est solubilisé dans du dichlorométhane (30ml). On ajoute du pyridinium chlorochromate (1,20g; 5,54mmoles)en solution dans 8ml de dichlorométhane. Le milieu réactionnel est agité pendant deux heures à température ambiante, puis le solvant évaporé.

Le produit est purifié par chromatographie sur colonne, élué par un mélange composé d'éther (80%) et d'hexane (20%). Les solvants sont évaporés et le solide obtenu est repris dans de l'hexane, filtré et séché. On obtient ainsi 920mg; (75%) d'aldéhyde p-(5,6,7,8-tétrahydro-5,5,8,8-tétraméthyl-2-napht [ 2,3-d ]oxazolyl) benzoïque, qui fond à 179°C.

EXEMPLE 13

Ethylamide de l'acide p-(5,6,7,8-tétrahydro-5,5,8,8-tétraméthyl-2-napht[ 2,3-d ]oxazolyl) benzoïque.

a) Chlorure de l'acide p-(5,6,7,8-tétrahydro-5,5,8,8-tétraméthyl-2-napht[ 2,3-d ]oxazolyl) benzoïque.

L'acide p-(5,6,7,8-tétrahydro-5,5,8,8-tétraméthyl-2-napht [ 2,3-d ]oxazolyl) benzoïque (4,57g; 13,1mmoles) est mis en suspension dans du dichlorométhane (200ml). On ajoute goutte à goutte de la dicyclohexylamine (2,37g; 13,1mmoles), puis évapore le dichlorométhane.

Le solide obtenu est repris dans de l'éther (500ml), filtré puis séché. On obtient ainsi le sel de dicyclohexylamine de l'acide p-(5,6,7,8-tétrahydro-5,5,8,8-tétraméthyl-2-napht[ 2,3-d ]oxazolyl) benzoïque (6,94g; 100%.

Le sel précédent est solubilisé dans du dichlorométhane (100ml). La solution obtenue est refroidie à 0°C, puis on ajoute goutte à goutte du chlorure de thionyle (1,55g; 13,1mmoles).

Le milieu réactionnel est agité pendant 2h à température ambiante. On filtre le chlorhydrate de dicyclohexylamine formé et évapore le dichlorométhane.

Le chlorure d'acide brut ainsi obtenu est utilisé tel quel pour la suite de la synthèse.

b) Ethylamide de l'acide p-(5,6,7,8-tétrahydro-5,5,8,8-tétraméthyl-2-napht[ 2,3-d ]oxazolyl) benzoïque.

De l'éthylamine (216mg; 4,8mmoles) est solubilisée dans du tétrahydrofuranne sec (25ml). On ajoute successivement de la triéthylamine (485mg; 4,8mmoles), puis goutte à goutte le chlorure d'acide obtenu en 13 a) (1,6g; 4,4mmoles) en solution dans du tétrahydrofuranne sec (25ml).

Le milieu réactionnel est agité pendant une heure à température ambiante puis versé dans de l'acide chlorhydrique 2N (200ml). Le produit est extrait par de l'éther (3x100ml), puis la phase organique est lavée à l'eau (3 fois), par une solution saturée de chlorure de sodium, et séchée sur sulfate de magnésium.

La solution est filtrée et les solvants évaporés.

Le solide obtenu est repris par de l'hexane (300ml) filtré et séché.

On obtient ainsi l'éthylamide de l'acide p-(5,6,7,8-tétrahydro-5,5,8,8-tétraméthyl-2-napht [ 2,3-d ]oxazolyl) benzoïque: 1,07g (65%), qui fond à 174°C.

EXEMPLE 14

Morpholide de l'acide p-(5,6,7,8-tétrahydro-5,5,8,8-tétraméthyl-2-napht[ 2,3-d ]oxazolyl) benzoïque.

De la morpholine (417mg; 4,8mmoles) est solubilisée dans du tétrahydrofuranne sec (25ml). On ajoute successivement de la triéthylamine (485mg; 4,8mmoles), puis goutte à goutte, le chlorure de l'acide

p-(5,6,7,8-tétrahydro-5,5,8,8-tétraméthyl-2-napht[ 2,3-d ]oxazolyl) benzoïque (1,6g; 4,4mmoles) en solution dans du tétrahydrofuranne sec (25ml).

Le milieu réactionnel est agité pendant deux heures à température ambiante, puis versé dans de l'acide chlorhydrique 4N (200ml).

Le produit est extrait par de l'éther (3x200ml), la phase organique est lavée par de l'eau (3 fois), puis par une solution saturée de chlorure de sodium et séchée sur sulfate de magnésium.

La solution est filtrée, les solvants évaporés et le produit purifié par chromatographie sur silice, élué par un mélange de dichlorométhane (50%), d'éther (20%) et d'hexane (30%).

Les solvants sont évaporés, et le solide obtenu est repris par de l'hexane (300ml).

On obtient ainsi le morpholide de l'acide p-(5, 6, 7, 8-tétrahydro-5, 5, 8, 8-tétraméthyl-2-napht [2, 3-d] oxazolyl) benzoïque : 1, 13g (62%), qui fond à 193°C.

EXAMPLE 15

Ester 2-hydroxyéthylique de L'acide p-(5, 6, 7, 8-tétrahydro-5, 5, 8, 8-tétraméthyl-2-napht [2, 3-d] oxazolyl) benzoïque.

De l'éthylène glycol (298mg; 4, 8mmoles) est solubilisé dans du dichlorométhane sec (25ml). On ajoute successivement de la pyridine: 380mg (4, 8mmoles), puis goutte à goutte le chlorure de l'acide p-(5, 6, 7, 8-tétrahydro-5, 5, 8, 8-tétraméthyl-2-napht [2, 3-d] oxazolyl) benzoïque (1, 6g; 4, 4mmoles), en solution dans du dichlorométhane sec (25ml).

Le milieu réactionnel est agité pendant deux heures à température ambiante, puis versé dans 200ml d'acide chlorhydrique 4N.

La produit est extrait par de l'éther (3x200ml). La phase organique est lavée par de l'eau (3 fois), puis par une solution saturée de chlorure de sodium, et séchée sur sulfate de magnésium.

La solution est filtrée et les solvants évaporés.

Le produit est purifié par chromatographie sur silice, élué par un mélange de dichlorométhane (70%) et d'éther (30%).

On obtient ainsi 875mg (51%) d'ester 2-hydroxyéthylique de l'acide p-(5, 6, 7, 8-tétrahydro-5, 5, 8, 8-tétraméthyl-2-napht [2, 3-d] oxazolyl) benzoïque, qui fond à 144°C.

EXAMPLE 16

Ester méthilique de l'acide p-(5, 6, 7, 8-tétrahydro-5, 5, 8, 8-téramethyl 2-napht [2, 3-d] imidazolyl) benzoïque.

a) 2, 3-dinitro-5, 6, 7, 8-tétrahydro-5, 5, 8, 8-tétraméthyl naphtalène.

91, 5g: (490mmoles) de 5, 6, 7, 8-tétrahydro-5, 5, 8, 8-tétraméthyl naphtalène sont solubilisés dans de l'acide sulfurique concentré (365ml). On refroidit le milieu à O°C et ajoute, sous agitation mécanique, de l'acide nitrique fumant (365ml).

On agite le milieu pendant 2h à température ambiante, puis on le verse sur de la glace.

On extrait le produit par de l'éther éthylique (3x1 litre). La phase organique est neutralisée par addition de bicarbonate de sodium solide (+300ml d'eau), puis décantée et séchée sur sulfate de magnésium.

La phase organique est filtrée, le solvant évaporé, et le solide obtenu, recristallisé dans le cyclohexane.

On obtient ainsi 80,19g (59%) de 2,3-dinitro-5,6,7,8-tétrahydro-5,5,8,8-tétraméthyl naphtalène, qui fond à 200°C.

b) 2,3-diamino-5,6,7,8-tétrahydro-5,5,8,8-tétraméthyl naphtalène.

(36,12g; 130mmoles) de 2,3-dinitro-5,6,7,8-tétrahydro-5,5,8,8-tétraméthyl naphtalène sont solubilisés dans du méthanol (1 1). On ajoute environ deux spatules de Nickel de Raney, lavé au méthanol, et hydrogène jusqu'à fin d'absorption. Le catalyseur est filtré. Les solvants évaporés et le solide obtenu, lavé par environ 300ml d'hexane.

On obtient ainsi: 14,49g (51%) de 2,3-diamino-5,6,7,8-tétrahydro-5,5,8,8-tétraméthyl naphtalène, qui fond à 185°C.

c) Ester méthylique de l'acide p-[(3-amino-(5,6,7,8-tétrahydro-5,5,8,8-tétraméthyl-2-naphtyl)carbamoyl]benzoïque.

Le 2,3-diamino-(5,6,7,8-tétrahydro-5,5,8,8-tétraméthyl naphtalène (6,55g; 30mmoles) est mélangé à de l'éther éthylique (170ml); on ajoute de la triéthylamine (4,2ml, 30mmoles), puis, goutte à goutte, du p-chloroformylbenzoate de méthyle (5,96g; 30mmoles) en solution dans de l'éther (70ml).

On agite 2h à température ambiante, puis verse le milieu réactionnel dans un mélange d'eau (400ml), et de dichlorométhane (400ml).

La phase organique est lavée par une solution saturée de bicarbonate de sodium, puis par une solution saturée de chlorure de sodium, séchée sur sulfate de magnésium, filtrée et les solvants évaporés.

Le solide obtenu est purifié par chromatographie sur colonne, élué par un système composé d'éther éthylique (10%) et de dichlorométhane (90%).

On obtient ainsi: 3,73g (33%) d'ester méthylique de l'acide p-[ (3-amino-(5,6,7,8-tétrahydro-5,5,8,8-tétraméthyl-2-naphtyl)carbamoyl] benzoïque, sous forme d'un solide blanc.

d) Ester méthylique de l'acide p-(5,6,7,8-tétrahydro-5,5,8,8-tétraméthyl-2-napht[ 2,3-d ]imidazolyl) benzoïque.

3,65g; (9,6mmoles) d'ester méthylique de l'acide p[(-3-amino-(5,6,7,8-tétrahydro-5,5,8,8-tétraméthyl-2-naphtyl)carbamoyl] benzoïque sont solubilisés dans du xylène (200ml). On ajoute de l'acide p.toluène sulfonique, monohydrate (1,82g; 9,6mmoles), et chauffe à reflux, sous agitation, pendant une heure. Le xylène est évaporé, on ajoute de l'eau (300ml), et 300ml d'une solution saturée de bicarbonate de sodium. La phase aqueuse est extraite par du dichlorométhane (3x300ml), et la phase organique est lavée par une solution saturée de chlorure de sodium. On sèche sur sulfate de magnésium, filtre, évapore les solvants, et purifie le solide obtenu par chromatographie sur colonne, éluée par un mélange composé de dichlorométhane (95%) et d'éther éthylique (5%).

On obtient ainsi: 2,7g (78%) d'ester méthylique de l'acide p-(5,6,7,8-trétrahydro-5,5,8,8-tétraméthyl-2-napht [2,3-d] imidazolyl) benzïque, qui fond à 270–275°C (décomposition).

<u>EXAMPLE 17</u>

<u>Acide p-(5,6,7,8-tétrahydro-5,5,8,8-tétraméthyl-2-napht [2,3-d] imidazolyl) benzoïque.</u>

1,52g; (4,2mmoles) d'ester méthylique de l'acide p-(5,6,7,8-tétrahydro-5,5,8,8-tétraméthyl-2-napht [2,3-d] imidazolyl) benzoïque est mélangé avec du méthanol (300ml).

On ajoute une solution de soude 5N (8,4ml), et chauffe à reflux pendant 24 h.

Le méthanol est évaporé, puis le pH ajusté à 5 par addition d'acide chlorhydrique 1 N.

On extrait le produit par de l'éther (5X400 ml), lave la phase organique à l'eau (2x500ml), puis par une solution saturée de chlorure de sodium (2X300 ml).

La phase organique est séchée sur sulfate de magnésium, filtrée, et les solvants évaporés.

On reprend le solide obtenu dans de l'hexane (300ml), filtre et sèche à l'étuve.

On obtient ainsi 920mg; (63%) d'acide p-(5,6,7,8-tétrahydro-5,5,8,8-tétraméthyl-2-napht [2,3-d] imidazolyl) benzoïque, qui fond à 250°C (décomposition).

<u>EXEMPLE 18</u>

<u>2-(4-méthyl)phényl-5,6,7,8-tétrahydro-5,5,8,8-tétraméthyl-napht [2,3-d] imidazole.</u>

a) N-(3-amino-5,6,7,8-tétrahydro-5,5,8,8-tétraméthyl-2-naphtyl)-4-méthylbenzamide.

4g (18mmoles) de 2,3-diamino-5,6,7,8-tétrahydro-5,5,8,8-tétraméthyl naphtalène sont mis en suspension dans de l'éther (100ml). On ajoute de la triéthylamine (2,55ml, 18,3mmoles), puis 2,83g; (18,3mmoles) de chlorure de méthyl-4 benzoyle en solution dans de l'éther (50ml).

On agite pendant 1h, ajouté de l'eau (300ml) et du dichlorométhane (300ml). La phase aqueuse est extraite par du dichlorométhane (2x300ml), et la phase organique lavée avec une solution saturée de chlorure de sodium.

On sèche la phase organique sur sulfate de magnésium, filtre et évapore les solvants.

Le produit est purifié par chromatographie sur colonne, élué par un mélange d'éther (10%) et de dichlorométhane (90%).

On obtient ainsi (2,75g; 45%) de N-(3-amino-5,6,7,8-tétrahydro-5,5,8,8-tétraméthyl-2-naphtyl)-4-méthylbenzamide, qui fond à 180°C.

b) 2-(4-méthyl)phényl-5,6,7,8-tétrahydro-5,5,8,8-tétraméthyl-napht [ 2,3-d ]imidazole.

(2,72g; 8,1mmoles) de N-(3-amino-5,6,7,8-tétrahydro-5,5,8,8-tétraméthyl-2-naphtyl)-4-méthylbenzamide sont mélangés avec du xylène (150ml). On ajoute de l'acide p.toluène sulfonique (1,54g; 8,1mmoles), et chauffe à reflux pendant 2h. Le xylène est évaporé, et on ajoute de l'eau (300ml), et du dichlorométhane (300ml).

La phase aqueuse est extraite par du dichlorométhane, la phase organique lavée par une solution saturée de chlorure de sodium, séchée sur du sulfate de magnésium, filtrée, et les solvants évaporés.

Le produit est purifié par chromatographie sur colonne, élué par un mélange d'éther (10%) et d'hexane (90%).

On obtient ainsi (1,78g; 69%) de 2-(4-méthyl)phényl-5,6,7,8-tétrahydro-5,5,8,8-tétraméthyl-napht[ 2,3-d ]imidazole.

EXEMPLE DE FORMULATIONS

A - Voie orale

1) Comprimé de 0,2 g
Acide p-(5,6,7,8-tétrahydro-5,5,8,8-tétraméthyl-2-naphto [2,3-b]thiényl) benzoïque 0,0001 g
Phosphate bicalcique 0,020 g
Silice 0,020 g
Lactose 0,030 g
Talc 0,010 g
Stéarate de magnésium 0,005 g
Amidon q.s.p. 0,200 g

Dans cet exemple, on peut remplacer le composé actif par son ester méthylique.

2) Capsule de 0,4g contenant:
Acide p-(5,6,7,8-tétrahydro-5,5,8,8-tétraméthyl-naphto [2,3-b ]furan-2-yl) benzoïque 0,0002g
Glycérine 0,200g
Saccharose 0,050g
Polyéthylène glycol 400 0,050g
Eau purifiée q.s.p.0,400g

La capsule est composée de gélatine, glycérine, dioxyde de titane et eau.

3) Gélule de 0,5g contenant:
Morpholide de l'acide p-(5,6,7,8-tétrahydro-5,5,8,8-tétraméthyl-2-napht[2,3-d]oxazolyl) benzoïque 0,0005 g
Amidon de maïs 0,150 g
Stéarate de magnésium 0,250 g
Saccharose q.s.p. 0,500 g

La poudre est conditionnée dans une gélule composée de gélatine et de $TiO_2$.

Voie topique

1) Onguent

a) Acide p-(5,6,7,8-tétrahydro-5,5,8,8-tétraméthyl-2-napht [2,3-d]oxazolyl) benzoïque 0,0001 g
Alcool stéarylique 3,000 g
Lanoline 5,000 g
Vaseline 15,000 g
Eau distillée q.s.p. 100,000 g

b) Acide p-(5,6,7,8-tétrahydro-5,5,8,8-tétraméthyl-2-1H-benz [ f ]indolyl) benzoïque 0,0005 g
Alcool stéarylique 3,000 g
Lanoline 5,000 g
Vaseline 15,000 g
Eaux distillée q.s.p. 100,000 g

2) Gel

Ester méthylique de l'acide p-(5,6,7,8-tétrahydro-5,5,8,8-tétraméthyl-naphto[ 2,3-b ]furan-2-yl) benzoïque 0,0005 g
Hydroxypropyl cellulose, vendue par la Société Hercules sous le nom de "Klucel HF" 2,000 g
Eau/éthanol (50:50) q.s.p. 100,000 g

**Revendications pour les Etats Contractants: AT, BE, CH, DE, FR, GB, IT, LI, NL, SE**

1. Composés hétérocycliques polycycliques, caractérisés par le fait qu'ils répondent à la formule générale suivante

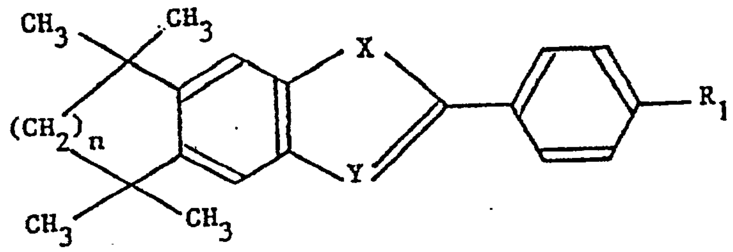

(I)

dans laquelle:
n est 1 ou 2
$R_1$ représente:

(i) un radical alkyle inférieur,

(ii) $-CH_2OH$

ou (iii) 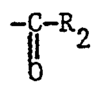

$R_2$ représentant:

(a) un atome d'hydrogène,

(b) le radical 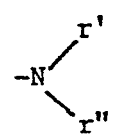

ou (c) le radical $-OR_3$, $R_3$ représentant un atome d'hydrogène, un radical alkyle ayant de 1 à 20 atomes de carbone, un radical mono ou polyhydroxyalkyle, un radical aryle ou aralkyle éventuellement substitué(s) ou un reste d'un sucre ou encore représente le radical:

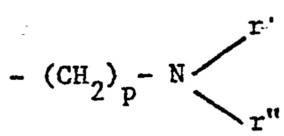

p étant 1, 2 ou 3,

r' et r" représentant un atome d'hydrogène, un radical alkyle inférieur, un radical monohydroxyalkyle ou polyhydroxyalkyle, un radical aryle éventuellement substitué, un reste d'aminoacide ou de sucre aminé ou encore pris ensemble forment un hétérocycle,
X représente un atome d'oxygène, un atome de soufre, SO, $SO_2$ ou le radical $-NR_4$,
et Y représente $CR_4$ ou un atome d'azote
$R_4$ représentant un atome d'hydrogène ou un radical alkyle inférieur,
et les sels desdits dérivés hétérocycliques polycycliques de formule (I).

2. Composés selon la revendication 1, caractérisés par le fait que le radical alkyle inférieur a de 1 à 4 atomes de carbone tels que les radicaux méthyle, éthyle, isopropyle, butyle et tertiobutyle.

3. Composés selon la revendication 1, caractérisés par le fait que le radical monohydroxyalkyle est un radical ayant de 2 à 4 atomes de carbone tels que les radicaux 2-hydroxyéthyle, 2-hydroxypropyle et 2'-hydroxy-2-éthoxy éthyle.

4. Composés selon la revendication 1, caractérisés par le fait que le radical polyhydroxyalkyle contient de 3 à 6 atomes de carbone et de 2 à 5 groupes hydroxyles tels que les radicaux 2,3-dihydroxypropyle, 2,3,4-trihydroxybutyle, 2,3,4,5-tétrahydroxypentyle ou le reste du pentaérythritol.

5. Composés selon la revendication 1, caractérisés par le fait que le radical aryle est un radical phényle éventuellement substitué par un halogène, un hydroxy, une fonction nitro ou un radical alkyle inférieur.

6. Composés selon la revendication 1, caractérisés par le fait que les radicaux r' et r'', pris ensemble, forment un hétérocyclique tels que les radicaux pipéridino, pipérazino, morpholino, pyrrolidino ou 4-(2'-hydroxyéthyl) pipérazino.

7. Composés selon l'une quelconque des revendications précédentes, caractérisés par le fait qu'ils correspondent à la formule générale suivante:

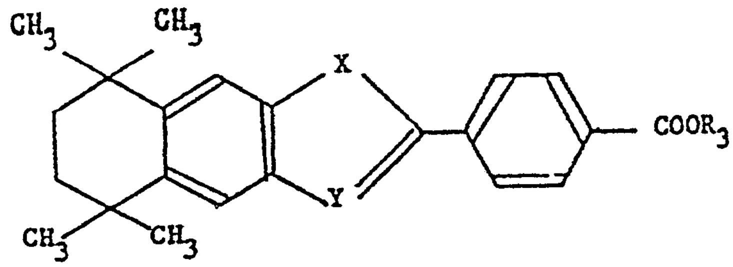

(II)

dans laquelle:
$R_3$ représente un atome d'hydrogène ou un radical alkyle inférieur,
X représente un atome d'oxygène, un atome de soufre ou le radical $NR_4$, et Y représente $CR_4$ ou un atome d'azote, $R_4$ représentant un atome d'hydrogène ou le radical méthyle.

8. Composés selon l'une quelconque des revendications précédentes, caractérisés par le fait qu'ils sont pris dans le groupe constitué par :
- l'ester méthylique de l'acide p-(5,6,7,8-tétrahydro-5,5,8,8-tétraméthyl-2-naphto[ 2,3-b ]furyl) benzoïque,
- l'acide p-(5,6,7,8-tétrahydro-5,5,8,8-tétraméthyl-2-naphto [ 2,3-b ]furyl) benzoïque,
- l'ester méthylique de l'acide p-(5,6,7,8-tétrahydro-5,5,8,8-tétraméthyl-2-naphto[ 2,3-b ]thiényl) benzoïque,
- l'acide p-(5,6,7,8-tétrahydro-5,5,8,8-tétraméthyl-2-naphto [ 2,3-b ]thiényl) benzoïque,
- l'acide p-(5,6,7,8-tétrahydro-5,5,8,8-tétraméthyl-2-1H-benz[ f ]indolyl) benzoïque,
- l'ester méthylique de l'acide p-(5,6,7,8-tétrahydro-5,5,8,8-tétraméthyl-2-1H-benz[ f ]indolyl) benzoïque,
- l'ester méthylique de l'acide p-(1-méthyl 5,6,7,8-tétrahydro-5,5,8,8-tétraméthyl-2-benz[ f ]indolyl) benzoïque,
- l'acide p-(1-méthyl 5,6,7,8-tétrahydro-5,5,8,8-tétraméthyl-2-benz[ f ]indolyl) benzoïque,
- l'ester méthylique de l'acide p-(5,6,7,8-tétrahydro-5,5,8,8-tétraméthyl-2 napht[ 2,3-d ]oxazolyl) benzoïque,
- l'acide p-(5,6,7,8-tétrahydro-5,5,8,8-tétraméthyl-2-napht [ 2,3-d ]oxazolyl) benzoïque,
- l'alcool p-(5,6,7,8-tétrahydro-5,5,8,8-tétraméthyl-2-napht [ 2,3-d ]oxazolyl) benzylique,
- l'aldéhyde p-(5,6,7,8-tétrahydro-5,5,8,8-tétraméthyl-2-napht [ 2,3-d ]oxazolyl benzoïque,
- l'éthylamide de l'acide p-(5,6,7,8-tétrahydro-5,5,8,8-tétraméthyl-2-napht[ 2,3-d ]oxazolyl) benzoïque,
- le morpholide de l'acide p-(5,6,7,8-tétrahydro-5,5,8,8-tétraméthyl-2-napht[ 2,3-d ]oxazolyl) benzoïque,
- l'ester 2-hydroxyéthylique de l'acide p-(5,6,7,8-tétrahydro-5,5,8,8-tétraméthyl-2-napht[ 2,3-d ]oxazolyl) benzoïque,
- l'ester méthylique de l'acide p-(5,6,7,8-tétrahydro-5,5,8,8-tétraméthyl-2-napht[ 2,3-d ]imidazolyl) benzoïque,
- l'acide p-(5,6,7,8-tétrahydro-5,5,8,8-tétraméthyl-2-napht [ 2,3-d ]imidazolyl) benzoïque, et
- le 2-(4-méthyl) phényl-5,6,7,8-tétrahydro-5,5,8,8-tétraméthyl-napht[ 2,3-d ]imidazole.

9. Procédé de préparation des composés hétérocycliques polycycliques selon l'une quelconque des revendications 1 à 8, caractérisé par le fait qu'il consiste à faire réagir un dérivé d'acide carboxylique aromatique de formule:

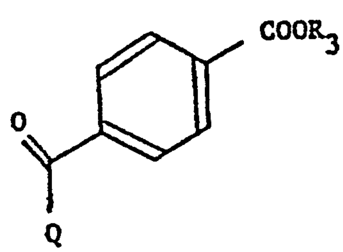

dans laquelle:
Q représente OH ou Cl et $R_3$ est tel que défini à la revendication 1 à l'exception d'un atome d'hydrogène,
(a) soit sur un dérivé aromatique diamino, hydroxyamino ou thioamino de formule:

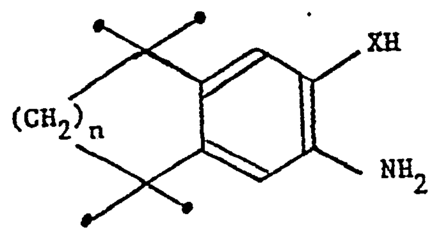

dans laquelle:
n est 1 ou 2 et X représente NH, O ou S
et à procéder à une réaction de cyclisation déshydratante en présence d'un catalyseur acide,
(b) soit sur un dérivé de formule:

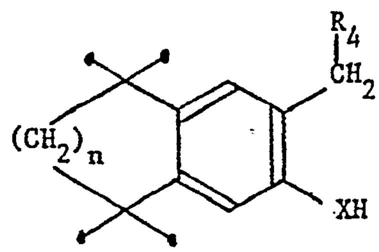

dans laquelle:
n est 1 ou 2, $R_4$ est tel que défini à la revendication 1 et X représente NH, O ou S,
et à procéder à une bromation du composé obtenu et ensuite à cycliser selon les conditions de la réaction de Wittig ou Wittig-Horner le dérivé intermédiaire porteur d'un groupement phosphonium ou phosphinyle,et éventuellement à transformer selon les méthodes connues, lesdits composés obtenus en vue d'obtenir l'une quelconque des autres significations du radical $R_1$.

10. Procédé selon la revendication 9, caractérisé par le fait que la réaction de cyclisation déshydratante est effectuée en présence d'un acide sulfonique tel que l'acide p-toluène sulfonique dans un solvant inerte tel que le toluène ou le xylène.

11. Procédé selon la revendication 9, caractérisé par le fait que la réaction de bromation est effectuée en présence de N-bromosuccinimide dans du benzène ou du tétrachlorure de carbone.

12. Composés intermédiaires de synthèse, caractérisés par le fait qu'ils répondent à la formule suivante:

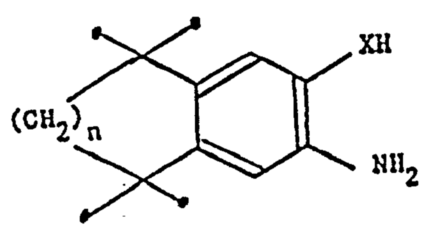

dans laquelle:
n est 1 ou 2,
et X représente un atome d'oxygène, un atome de soufre ou le radical $NR_4$, $R_4$ représentant un radical alkyle inférieur.

13. Composés selon la revendication 12, caractérisés par le fait qu'il est:
– le 3-amino-5,6,7,8-tétrahydro-5,5,8,8-tétraméthyl-2-naphtol.

14. Médicament, caractérisé par le fait qu'il est une composé de formule (I) selon l'une quelconque des revendications 1 à 8.

15. Composition pharmaceutique, caractérisée par le fait qu'elle contient dans un véhicule approprié pour une administration par voie entérale, parentérale, rectale, topique ou oculaire au moins un composé de formule (I) selon l'une quelconque des revendications 1 à 8.

16. Composition selon la revendication 15, caractérisée par le fait qu'elle est administrée à une dose

journalière de 0,01µg/Kg à 1 mg/kg de poids corporel.

17. Composition selon la revendication 16, caractérisée par le fait qu'elle se présente sous une forme appropriée pour une application topique et contient le composé actif de formule (I) à une concentration comprise entre 0,00001 à 0,01% en poids.

18. Utilisation d'un composé selon l'une quelconque des revendications 1 à 8, pour la préparation d'une composition pharmaceutique destiné au traitement des affections dermatoligiques, rhumatismales, respiratoires ainsi qu'ophtalmologiques.

19. Composition cosmétique pour l^hygiène corporelle et capillaire, caractérisée par le fait qu'elle contient, dans un véhicule cosmétique approprié, au moins un composé de formule (I) selon l'une quelconque des revendications 1 à 8.

20. Composition cosmétique selon la revendication 19, caractérisée par le fait qu'elle contient le composé de formule (I) à une concentration comprise entre 0,00001 et 0,01% en poids.

**Revendications pour les Etats Contractants: ES, GR**

1. Procédé de préparation des composés hétérocycliques polycycliques selon la formule générale suivante:

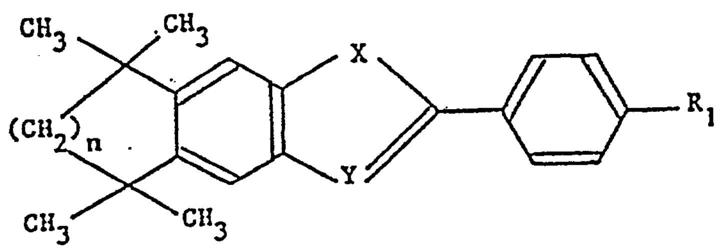

(I)

dans laquelle n est 1 ou 2, $R_1$ représente: (i) un radical alkyle inférieur, (ii) $-CH_2OH$ ou (iii)

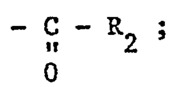

$R_2$ représentant: (a) un atome d'hydrogène, (b) le radical

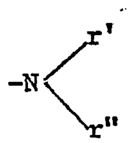

ou (c) le radical $-OR_3$, $R_3$ réprésentant un atome d'hydrogène, un radical alkyle ayant 1 à 20 atomes de carbone, un radical mono ou polyhydroxyalkyle, un radical aryle ou aralkyle éventuellement substitué(s) ou un reste d'un sucre ou encore représente le radical:

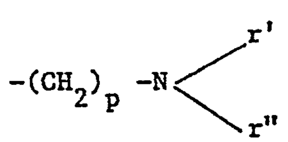

p étant 1, 2 ou 3, r' et r'' représentant un atome d'hydrogène, un radical alkyle inférieur, un radical monohydroxyalkyle ou polyhydroxyalkyle, un radical aryle éventuellement substitué, un reste d'aminoacide ou de sucre aminé ou encore pris ensemble forment un hétérocycle, X représente un atome d'oxygène, un atome de soufre, SO, $SO_2$ ou le radical $-NR_4$ et Y représente $CR_4$ ou un atome d'azote

R4 représentant un atome d'hydrogène ou un radical alkyle inférieur, et les sels desdits dérivés hétérocycliques de formule (I), caractérisé par le fait qu'il consiste à faire réagir un dérivé carboxylique aromatique de formule:

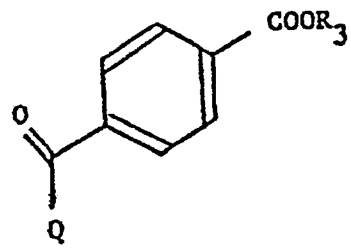

dans laquelle:
Q représente OH ou C1 et $R_3$ est tel que défini ci-dessus à l'exception d'un atome d'hydrogène,
(a) soit sur un dérivé aromatique diamino, hydroxyamino ou thioamino de formule:

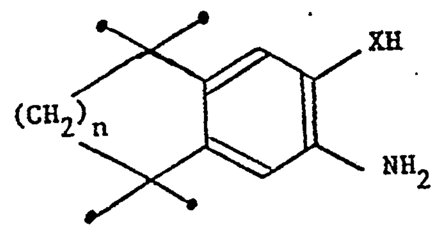

dans laquelle:
n est 1 ou 2 et X représente NH, O ou S
et à procéder à une réaction de cyclisation déshydratante en présence d'un catalyseur acide,
(b) soit sur un dérivé de formule:

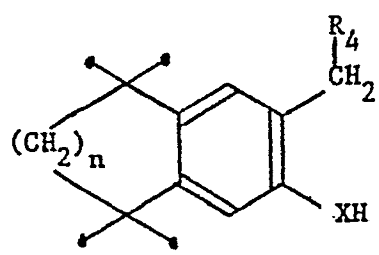

dans laquelle:
n est 1 ou 2, $R_4$ est tel que défini à la revendication 1 et X représente NH, O ou S,
et à procéder à une bromation du composé obtenu et ensuite à cycliser selon les conditions de la réaction de Wittig ou Wittig-Horner le dérivé intermédiaire porteur d'un groupement phosphonium ou phosphinyle, et éventuellement à transformer selon les méthodes connues, lesdits composés obtenus en vue d'obtenir l'une quelconque des autres significations du radical $R_1$.

2. Procédé selon la revendication 1, caractérisé par le fait que la réaction de cyclisation, déshydratante est effectuée en présence d'un acide sulfonique tel que l'acide p-toluène sulfonique dans un solvant inerte tel que le toluène ou le xylène.
3. Procédé selon la revendication 1, caractérisé par le fait que la réaction de bromation est effectuée en présence de N-bromosuccinimide dans du benzène ou du tétrachlorure de carbone.
4. Composition cosmétique pour l'hygiène corporelle et capillaire, caractérisée par le fait qu'elle contient, dans un véhicule cosmétique approprié, au moins un composé de formule (I) selon la revendication 1.
5. Composition cosmétique selon la revendication 4, caractérisée par le fait qu'elle contient le composé de formule (I) à une concentration comprise entre 0,00001 et 0,01% en poids.

**Patentansprüche für die Vertragsstaaten: AT, BE, CH, DE, FR, GB, IT, LI, NL, SE**

1. Heterocyclische polycyclische Verbindungen, dadurch gekennzeichnet, daß sie die folgende allgemeine Formel aufweisen

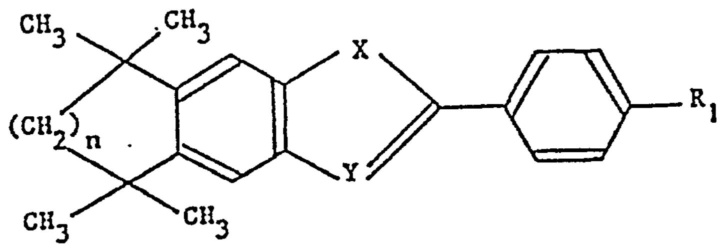 (I)

in der
n 1 oder 2 ist,
$R_1$ (i) ein Niederalkylrest, (ii) $-CH_2OH$ ou (iii)

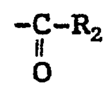

ist,
wobei $R_2$ (a) ein Wasserstoffatom, (b) der Rest

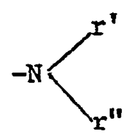

(c) der Rest -OR3 ist, wobei R3 ein Wasserstoffatom, ein Alkylrest mit 1 bis 20 Kohlenstoffatomen, ein Mono- oder Polyhydroxyalkylrest, ein gegebenenfalls substituierter Arylrest oder Aralkylrest oder der Rest eines Zuckers oder auch noch ein Rest der Formel

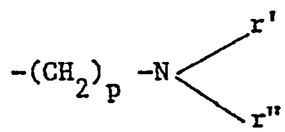

ist,
p 1, 2 oder 3 ist,
r′ und r″ ein Wasserstoffatom, ein Niederalkylrest, ein Monohydroxyalkyl- oder Polyhydroxyalkylrest, ein gegebenenfalls substituierter Arylrest, ein Aminosäurerest oder Aminozuckerrest sind oder auch gemeinsam einen heterocyclischen Ring bilden,
X ein Sauerstoffatom, ein Schwefelatom, SO, SO2 oder der Rest -NR4 ist, und
Y CR4 oder ein Stickstoffatom ist, wobei R4 ein Wasserstoffatom oder ein Niederalkylrest ist,
und die Salze dieser heterocyclischen polycyclischen Derivate der Formel (I).

2. Verbindungen nach Anspruch 1, dadurch gekennzeichnet, daß der Niederalkylrest 1 bis 4 Kohlenstoffatome aufweist, wie beispielsweise die Reste Methyl, Ethyl, Isopropyl, Butyl und tert. Butyl.

3. Verbindungen nach Anspruch 1, dadurch gekennzeichnet, daß der Monohydroxyalkylrest ein Rest mit 2 bis 4 Kohlenstoffatomen ist, wie beispielsweise die Reste 2-Hydroxyethyl, 2-Hydroxypropyl und 2′-Hydroxy-2-ethoxyethyl.

4. Verbindungen nach Anspruch 1, dadurch gekennzeichnet, daß der Polyhydroxyalkylrest 3 bis 6 Kohlenstoffatome und 2 bis 5 Hydroxygruppen enthält, wie beispielsweise die Reste 2,3-Dilhydroxypropyl, 2,3,4-Trihydroxybutyl, 2,3,4,5-Tetrahydroxypentyl oder der Pentaerythrit-Rest.

5. Verbindungen nach Anspruch 1, dadurch gekennzeichnet, daß der Arylrest ein Phenylrest ist, der gegebenenfalls durch ein Halogen, ein Hydroxygruppe, eine Nitrofunktion oder einen Niederalkylrest substituiert ist.

6. Verbindungen nach Anspruch 1, dadurch gekennzeichnet, daß die Reste r′ und r″ zusammengenommen einen heterocyclischen Ring bilden, wie beispielsweise die Piperidino-, Piperazino-, Morpholino-, Pyrrolidino- oder 4-(2′-Hydroxyethyl-)piperazino-Reste.

7. Verbindungen nach irgendeinem der vorangehenden Ansprüche, dadurch gekennzeichnet, daß sie die folgenden allgemeinen Formel entsprechen

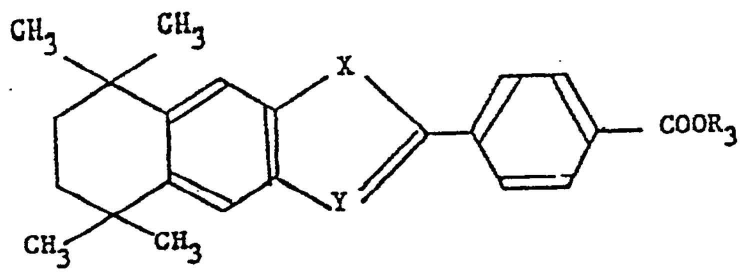

(II)

in der

R3 ein Wasserstoffatom oder ein Niederalkylrest ist,

X ein Sauerstoffatom, ein Schwefelatom oder der Rest NR4 ist, und

Y CR4 oder ein Stickstoffatom ist, wobei R4 ein Wasserstoffatom oder ein , Methylrest ist.

8. Verbindungen nach irgendeinem der vorangehenden Ansprüche gekennzeichnet, daß sie aus der aus folgenden Verbindungen gebildeten Gruppe stammen:

– p-(5,6,7,8-Tetrahydro-5,5,8,8-tetramethyl-2-naphthol[2,3-b]-benzoesäuremethylester;
– p-(5,6,7,8-Tetrahydro-5,5,8,8-tetramethyl-2-naphthol[2,3-b]furyl)-benzoesäure;
– p-(5,6,7,8-Tetrahydro-5,5,8,8-tetramethyl-2-naphthol[2,3-b]thienyl)-benzoesäuremethylester;
– p-(5,6,7,8-Tetrahydro-5,5,8,8-tetramethyl-2-naphthol[2,3-b]thienyl)-benzoesäure;
– p-(5,6,7,8-Tetrahydro-5,5,8,8-tetramethyl-2-1H-benz[f]-indolyl)-benzoesäure;
– p-(5,6,7,8-Tetrahydro-5,5,8,8.-tetramethyl-2-1H-benz[f]indolyl)-benzoesäureremethylester;
– p-(1-Methyl-5,6,7,8-tetrahydro-5,5,8,8-tetramethyl-2-benz[f]indolyl)-benzoesäuremethylester;
– p-(1-Methyl-5,6,7,8-tetrahydro--5,5,8,8-tetramethyl-2-benz[f]indolyl)-benzoesäure;
– p-(5,6,7,8-Tetrahydro-5,5,8,8-tetramethyl-2-naphth[2,3-d]oxazolyl)-benzoesäuremethylester;
– p-(5,6,7,8-Tetrahydro-5,5,8,8-tetramethyl-2-naphth[2,3-d]oxazolyl)-benzoesäure;
– p-(5,6,7,8-Tetrahydro-5,5,8,8-tetramethyl-2-naphth[2,3-d]oxazolyl)-benzylalkohol;
– p-(5,6,7,8-Tetrahydro-5,5,8,8-tetramethyl-2-naphth[2,3-d]oxazolyl)-benzaldehyd;
– p-(5,6,7,8-Tetrahydro-5,5,8,8-tetramethyl-2-naphth[2,3-d]oxazolyl)-benzoesäureethylamid;
– p-(5,6,7,8-Tetrahydro-5,5,8,8-tetramethyl-2-naphth[2,3-d]oxazolyl)-benzoesäuremorpholid;
– p-(5,6,7,8-Tetrahydro-5,5,8,8-tetramethyl-2-naphth[2,3-d]oxazolyl)-benzoesäure-2-hydroxyethylether;
– p-(5,6,7,8-Tetrahydro-5,5,8,8-tetramethyl-2-naphth[2,3-d]imidazolyl)-benzoesäuremethylester;
– p-(5,6,7,8-Tetrahydro-5,5,8,8-tetramethyl-2-naphth[2,3-d]imidazolyl)-benzoesäure; und
– 2-(4-Methyl-)phenyl-5,6,7,8-tetrahydro-5,5,8,8-tetramthyl-naphth-[2,3-d]imidazol.

9. Verfahren zur Herstellung von heterocyclischen polycyclischen Verbindungen nach irgendeinem der Patentansprüche 1 bis 8, dadurch gekennzeichnet, daß es daraus besteht, ein Derivat einer aromatischen Carbonsäure der Formel

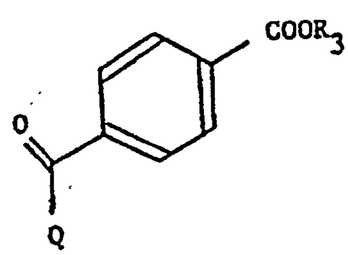

in der

Q OH der Cl ist und R3 in der Weise wie in Anspruch 1 definiert ist, mit Ausnahme der Bedeutung eines Wasserstoffatoms,

(a) entweder mit einem aromatischen Diamino-, Hydroxyamino- oder Thioamino-Derivat der Formel

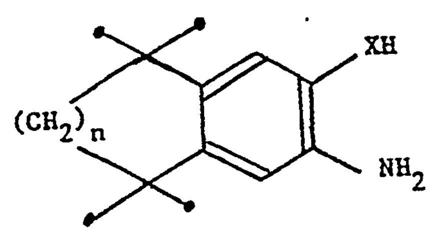

umzusetzen, in der

n 1 oder 2 ist und X NH, O oder S ist,

und danach eine dehydratisierende Cyclisierungsreaktion in Gegenwart eines sauren Katalysators durchzuführen; oder

(b) mit einem Derivat der Formel

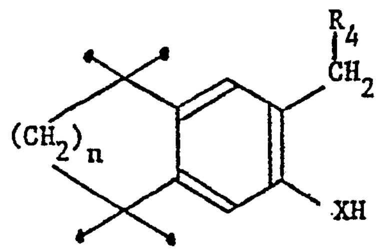

umzusetzen, in der

n 1 oder 2 ist, R4 in der Weise wie in Anspruch 1 definiert ist und X NH, O oder S ist,

und danach eine Bromierung der erhaltenen der erhaltenen Verbindung durchzuführen und das Zwischenprodukt, das eine Phosphonium- oder Phosphinylgruppe trägt, anschließend unter Bedingungen einer Bedingungen einer Wittig-Reaktion oder Wittig-Reaktion oder Wittig-Horner-Reaktion zu cyclisieren und gegenbenenfalls die erhaltenen Verbindungen nach bekannten Methoden in der Weise umzuwandeln, daß man Verbindungen mit irgendeiner anderen Bedeutung des Restes R1 erhält.

10. Verfahren nach Anspruch 9, dadurch gekennzeichnet, daß die dehydratisierende Cyclisierungsreaktion in Gegenwart einer Sulfonsäure wie beispielsweise p-Toluolsulfonsäure in einem inerten Lösungsmittel wie beispielsweise Toluol oder Ylol durchgeführt wird.

11. Verfahren nach Anspruch 9, dadurch gekennzeichnet, daß die Bromierungsreaktion in Gegenwart von N-Bromsuccinimid in Benzol oder Tetrachlorkohlenstoff durchgeführt wird.

12. Zwischenverbindungen der Synthese, dadurch gekennzeichnet, daß sie die folgende Formel aufweisen:

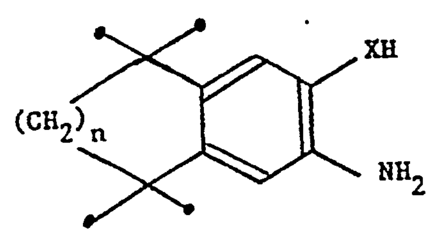

in der

n 1 oder 2 ist, und

X ein Sauerstoffatom, ein Schwefelatom, oder der Rest NR4 ist, wobei R4 ein Nideralkylrest ist.

13. Verbindungen nach Anspruch 12, dadurch gekennzeichnet, daß es die Verbindung 3-Amino-5,6,7,8-tetrahydro-5,5,8,8-tetramethyl-2-naphthol ist.

14. Arzneimittel, dadurch gekennzeichnet, daß es eine Verbindung der Formel (I) gemäß irgendeinem der Patentansprüche 1 bis 8 ist.

15. Pharmazeutische Zusammensetzung, dadurch gekennzeichnet, daß sie in einem Träger, der für die Verabreichung auf enteralem, parenteralem, rektalem, topischem oder okulärem Weg geeignet ist, wenigstens eine Verbindung der Formel (I) gemäß irgendeinem der Patentansprüche 1 bis 8 enthält.

16. Zusammensetzung nach Anspruch 15, dadurch gekennzeichnet, daß sie in einer täglichen Dosis von 0,01 µg bis 1 mg pro kg Körpergewicht verabreicht wird.

17. Zusammensetzung nach Anspruch 16, dadurch gekennzeichnet, daß sie in einer Form vorliegt, die für die topische Anwendung geeignet ist, und die aktive Verbindung der Formel (I) in einer Konzentration zwischen 0,00001 bis 0.01 Gew.-% enthält.

18. Verwendung einer Verbindung gemäß irgendeinem der Patentansprüche 1 bis 8 zur Herstellung einer pharmazeutischen Zusammensetzung, die für die Behandlung von dermatologischen Erkrankungen, rheumatischen Erkrankungen, Erkrankungen der Atmungsorgane sowie ophthalmologischen Erkrankungen bestimmt ist.

19. Kosmetische Zusammensetzung für die Körper- und Haarpflege, dadurch gekennzeichnet, daß sie in einem geeigneten kosmetischen Träger wenigstens eine Verbindung der Formel (I) gemäß irgendeinem der Patentansprüche 1 bis 8 enthält.

20. Kosmethische Zusammentzung nach Anspruch 19, dadurch gekennzeichnet, daß sie die Verbindung der Formel (I) in einer Konzentration zwischen 0,00001 und 0,01 Gew.-% enthält.

**Patentansprüche für die Vertragsstaaten: ES, GR**

1. Verfahren zur Herstellung heterocyclischer polycyclischer Verbindungen der folgenden allgemeinen Formel

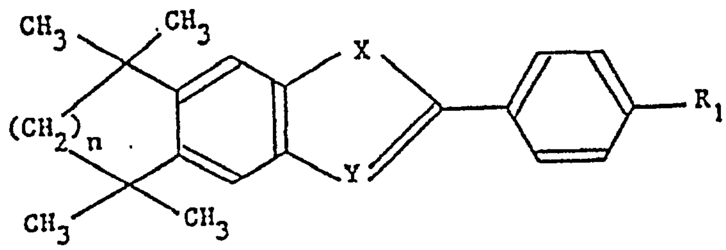

(I)

in der

n 1 oder 2 ist,

$R_1$ (i) ein Niederalkylrest, (ii) $-CH_2OH$ ou (iii)

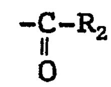

ist,

wobei $R_2$ (a) ein Wasserstoffatom, (b) der Rest

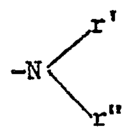

oder

(c) der Rest –OR3 ist, wobei $R_3$ ein Wasserstoffatom, ein Alkylrest mit 1 bis 20 Kohlenstoffatomen, ein Mono- oder Polyhydroxyalkylrest, ein gegebenenfalls substituierter Arylrest oder Aralkylrest oder der Rest eines Zuckers oder auch noch ein Rest der Formel

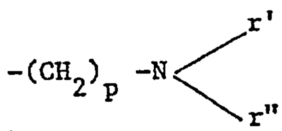

p 1, 2 oder 3 ist,

r', und r" ein Wasserstoffatom, ein Niederalkylrest, ein Monohydroxyalkyl- oder Polyhydroxyalkylrest, ein gegebenenfalls substituierter Arylrest, ein Aminosäurerest oder Aminozuckerrest sind oder auch gemeinsam einen heterocyclischen Ring bilden,

X ein Sauerstoffatom, ein Schwefelatom, SO, $SO_2$ oder der Rest $-NR_4$ ist, und

Y $CR_4$ oder ein Stickstoffatom ist, wobei $R_4$ ein Wasserstoffatom oder ein Niederalkylrest ist,

und der Salze dieser heterocyclischen Derivate der Formel (I), dadurch gekennzeichnet, daß es daraus besteht, ein Derivat einer aromatischen Carbonsäure der Formel

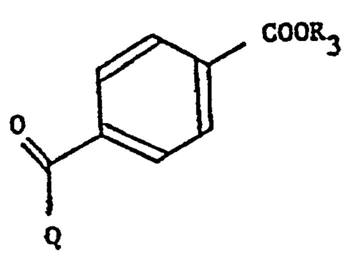

in derQ OH oder Cl ist und $R_3$ in der Weise wie oben definiert ist, mit Ausnahme der Bedeutung eines Wasserstoffatoms,

(a) entweder mit einem aromatischen Diamino-, Hydroxyamino- oder Thioamino-Derivat der Formel

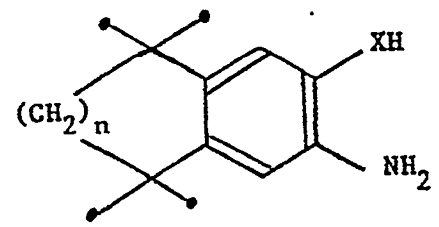

umzusetzen, in der n 1 oder 2 ist und X NH, O oder S ist,

und danach eine dehydratisierende Cyclisierungsreaktion in Gegenwart eines sauren Katalysators durchzuführen; oder

(b) mit einem Derivat der Formel

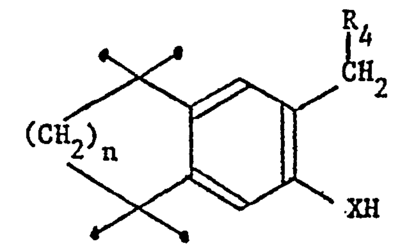

umzusetzen, in der

n 1 oder 2 ist, $R_4$ in der Weise wie in Anspruch 1 definiert ist und X NH, O oder S ist,

und danach eine Bromierung der erhaltenen Verbindung durchzuführen und das Zwischenprodukt, das eine Phosphonium- oder Phosphinylgruppe trägt, anschließend unter Bedingungen einer Wittig-Reaktion oder Wittig-Horner-Reaktion zu cyclisieren und gegebenenfalls die erhaltenen Verbindungen nach bekannten Methoden in der Weise umzuwandeln, daß man Verbindungen mit irgendeiner anderen Bedeutung des Restes $R_1$ erhält.

2. Verfahren nach Anspruch 1, dadurch gekennzeichnet, daß die dehydratisierende Cyclisierungsreaktion in Gegenwart einer Sulfonsäure wie beispielsweise p-Toluolsulfonsäure in einem inerten Lösungsmittel wie beispielsweise Toluol oder Xylol durchgeführt wird.

3. Verfahren nach Anspruch 1, dadurch gekennzeichnet, daß die Bromierungsreaktion in Gegenwart von N-Bromsuccinimid in Benzol oder Tetrachlorkohlenstoff durchgeführt wird.

4. Kosmetische Zusammensetzung für die Körper- und Haarpflege, dadurch gekennzeichnet, daß sie in einem geeigneten kosmetischen Träger wenigstens eine Verbindung der Formel (I) gemäß Patentanspruch 1 enthält.

5. Kosmetische Zusammensetzung nach Anspruch 4, dadurch gekennzeichnet, daß sie die Verbindung der Formel (I) in einer Konzentration zwischen 0,00001 und 0,01 Gew.-% enthält.

**Claims for the Contracting States: AT, BE, CH, DE, FR, GB, IT, LI, NL, SE**

1. Polycyclic heterocyclic compounds, characterized in that they correspond to the following general formula

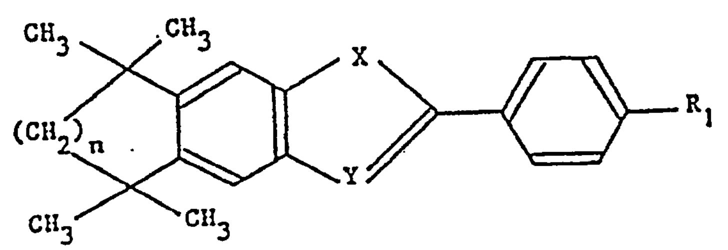

(I)

in which:

n is 1 or 2

$R_1$ represents:

(i) a lower alkyl radical,

(ii) $-CH_2OH$ or

(iii)

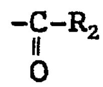

$R_2$ representing:
(a) a hydrogen atom,
(b) a radical

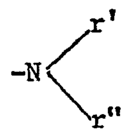

or (c) a radical $-OR_3$, $R_3$ representing a hydrogen atom, an alkyl radical having from 1 to 20 carbon atoms, a mono- or polyhydroxyalkyl radical, an optionally substituted aryl or aralkyl radical or a sugar residue, or alternatively represents a radical:

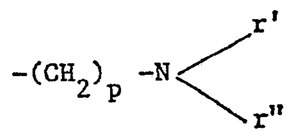

p being 1, 2 or 3,
r′ and r″ representing a hydrogen atom, a lower alkyl radical, a monohydroxyalkyl or polyhydroxyalkyl radical, an optionally substituted aryl radical, an amino acid residue or an amino sugar residue, or alternatively, taken together, form a heterocycle,
X represents an oxygen atom, a sulphur atom, SO, $SO_2$ or a radical $-NR_4$, and
Y represents $CR_4$ or a nitrogen atom
$R_4$ representing a hydrogen atom or a lower alkyl radical,
and the salts of the said polycyclic heterocyclic derivatives of formula (I).

2. Compounds according to Claim 1, characterized in that the lower alkyl radical has from 1 to 4 carbon atoms, such as methyl, ethyl, isopropyl, butyl and tertbutyl radicals.

3. Compounds according to Claim 1, characterized in that the monohydroxyalkyl radical is a radical having from 2 to 4 carbon atoms, such as 2-hydroxyethyl, 2-hydroxypropyl and 2′-hydroxy-2-ethoxyethyl radicals.

4. Compounds according to Claim 1, characterized in that the polyhydroxyalkyl radical contains from 3 to 6 carbon atoms and from 2 to 5 hydroxyl groups, such as 2,3-dihydroxypropyl, 2,3,4-trihydroxybutyl and 2,3,4,5 tetrahydroxypentyl radicals or the pentaerythritol residue.

5. Compounds according to Claim 1, characterized in that the aryl radical is a phenyl radical optionally substituted with a halogen, a hydroxy, a nitro group or a lower alkyl radical.

6. Compounds according to Claim 1, characterized in that the radicals r′ and r″, taken together, form a heterocycle such as piperidino, piperazino, morpholino, pyrrolidino or 4-(2″-hydroxyethyl)piperazino radicals.

7. Compounds according to any one of the preceding claims, characterized in that they correspond to the following general formula:

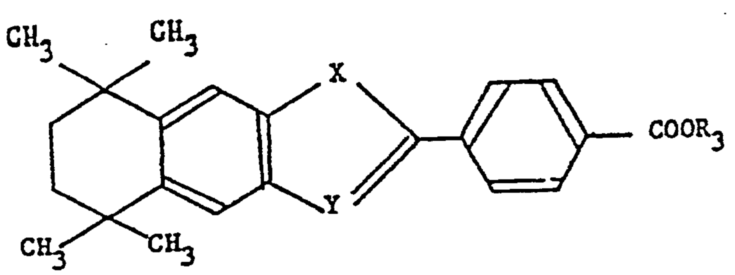

(II)

in which:
$R_3$ represents a hydrogen atom or a lower alkyl radical,
X represents an oxygen atom, a sulphur atom or a radical $NR_4$, and
Y represents $CR_4$ or a nitrogen atom, $R_4$ representing a hydrogen atom or a methyl radical.

8. Compounds according to any one of the preceding claims, characterized in that they are selected

from the group consisting of:
p-(5,6,7,8-tetrahydro-5,5,8,8-tetramethyl-2-naphtho[2,3-b]furyl)benzoic acid methyl ester,
p-(5,6,7,8-tetrahydro-5,5,8,8-tetramethyl-2-naphtho[2,3-b]furyl)benzoic acid,
p-(5,6,7,8-tetrahydro-5,5,8,8-tetramethyl-2-naphtho[2,3-b]thienyl)benzoic acid methyl ester,
p-(5,6,7,8-tetrahydro-5,5,8,8-tetramethyl-2-naphtho[2,3-b]thienyl)benzoic acid,
p-(5,6,7,8-tetrahydro-5,5,8,8-tetramethyl-2-1H-benz[f]indolyl)benzoic acid,
p-(5,6,7,8-tetrahydro-5,5,8,8-tetramethyl-2-1H-benz[f]indolyl)benzoic acid methyl ester,
p-(1-methyl-5,6,7,8-tetrahydro-5,5,8,8-tetramethyl-2-benz[f]indolyl)benzoic acid methyl ester,
p-(1-methyl-5,6,7,8-tetrahydro-5,5,8,8-tetramethyl-2-benz[f]indolyl)benzoic acid,
p-(5,6,7,8-tetrahydro-5,5,8,8-tetramethyl-2-naphth[2,3-d]oxazolyl)benzoic acid methyl ester,
p-(5,6,7,8-tetrahydro-5,5,8,8-tetramethyl-2-naphth[2,3-d]oxazolyl)benzoic acid,
p-(5,6,7,8-tetrahydro-5,5,8,8-tetramethyl-2-naphth[2,3-d]oxazolyl)benzoic alcohol,
p-(5,6,7,8-tetrahydro-5,5,8,8-tetramethyl-2-naphth[2,3-d]oxazolyl)benzaldehyde,
p-(5,6,7,8-tetrahydro-5,5,8,8-tetramethyl-2-naphth[2,3-d]oxazolyl)benzoic acid ethylamide,
p-(5,6,7,8-tetrahydro-5,5,8,8-tetramethyl-2-naphth[2,3-d]oxazolyl)benzoic acid morpholide,
p-(5,6,7,8-tetrahydro-5,5,8,8-tetramethyl-2-naphth[2,3-d]oxazolyl)benzoic acid 2-hydroxyethyl ester,
p-(5,6,7,8-tetrahydro-5,5,8,8-tetramethyl-2-naphth[2,3-d]imidazolyl)benzoic acid methyl ester,
p-(5,6,7,8-tetrahydro-5,5,8,8-tetramethyl-2-naphth[2,3-d]imidazolyl)benzoic acid, and
2-(4-methylphenyl)-5,6,7,8-tetrahydro-5,5,8,8-tetramethylnaphth[2,3-d]imidazole.

9. Process for preparing the polycyclic heterocyclic compounds according to any one of Claims 1 to 8, characterized in that it consists in reacting an aromatic carboxylic acid derivative of formula:

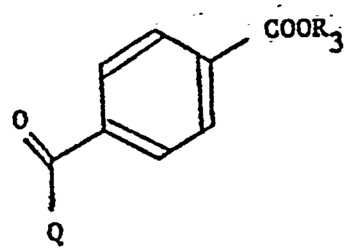

in which:
Q represents OH or Cl and $R_3$ is as defined in Claim 1 with the exception of a hydrogen atom,
(a) either with a diamino, hydroxyamino or thioamino aromatic derivative of formula:

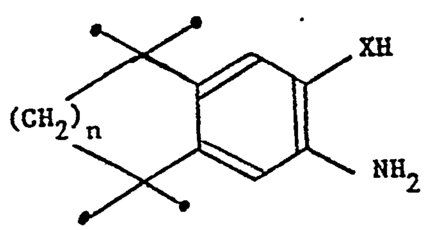

in which:
n is 1 or 2 and X represents NH, O or S and in performing a dehydrating cyclization reaction in the presence of an acid catalyst,
(b) or with a derivative of formula:

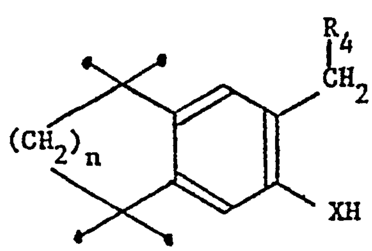

in which:
n is 1 or 2, $R_4$ is as defined in Claim 1 and X represents NH, O or S, and in performing a bromination of the compound obtained and then in cyclizing the intermediate derivative bearing
a phosphonium or phosphinyl group according to the conditions of the Wittig or Wittig-Horner reaction

and, where appropriate, in converting the said compounds obtained, according to known methods, for the purpose of obtaining any one of the other meanings of the radical $R_1$.

10. Process according to Claim 9, characterized in that the dehydrating cyclization reaction is performed in the presence of a sulphonic acid such as p-toluene-sulphonic acid in an inert solvent such as toluene or xylene.

11. Process according to Claim 9, characterized in that the bromination reaction is performed in the presence of N-bromosuccinimide in benzene or carbon tetrachloride.

12. Compounds which are synthesis intermediates, characterized in that they correspond to the following formula:

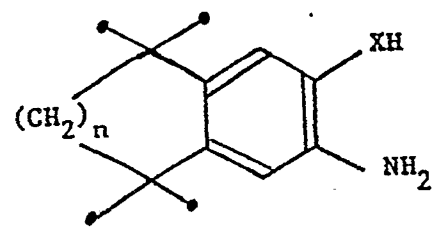

in which:

n is 1 or 2, and X represents an oxygen atom, a sulphur atom or a radical $NR_4$, $R_4$ representing a lower alkyl radical.

13. Compound according to Claim 12, characterized in that it is:

– 3-amino-5,6,7,8-tetrahydro-5,5,8,8-tetramethyl-2-naphthol.

14. Medicinal product, characterized in that it is a compound of formula (I) according to any one of Claims 1 to 8.

15. Pharmaceutical composition, characterized in that it contains at least one compound of formula (I) according to any one of Claims 1 to 8, in a vehicle suitable for enteral, parenteral, rectal, topical or ocular administration.

16. Composition according to Claim 15, characterized in that it is administered at a daily dose of 0.01 µg/kg to 1 mg/kg of body weight.

17. Composition according to Claim 16, characterized in that it is presented in a form suitable for topical administration and contains the active compound of formula (I) at a concentration of between 0.00001 and 0.01% by weight.

18. Use of a compound according to any one of Claims 1 to 8, for the preparation of a pharmaceutical composition intended for the treatment of dermatological, rheumatic, respiratory and also ophthalmological conditions.

19. Cosmetic composition for body and hair hygiene, characterized in that it contains at least one compound of formula (I) according to any one of Claims 1 to 8, in a suitable cosmetic vehicle.

20. Cosmetic composition according to Claim 19, characterized in that it contains the compound of formula (I) at a concentration of between 0.00001 and 0.01% by weight.

**Claims for the Contracting States: ES, GR**

1. Process for preparing the polycyclic heterocyclic compounds according to the following general formula:

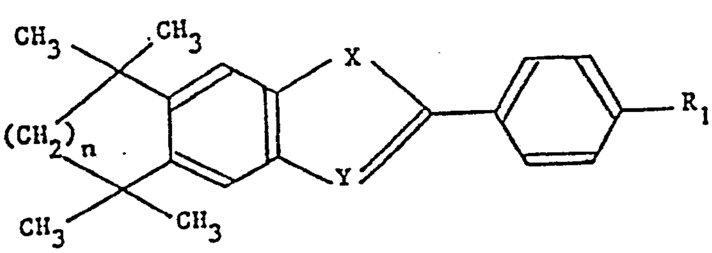

(I)

in which n is 1 or 2, $R_1$ represents: (i) a lower alkyl radical, (ii) $-CH_2OH$ or (iii)

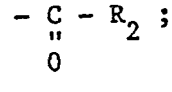

$R_2$ representing: (a) a hydrogen atom, (b) a radical

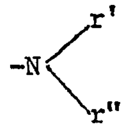

or (c) a radical $-OR_3$, $R_3$ representing a hydrogen atom, an alkyl radical having from 1 to 20 carbon atoms, a mono- or polyhydroxyalkyl radical, an optionally substituted aryl or aralkyl radical or a sugar residue, or alternatively represents a radical:

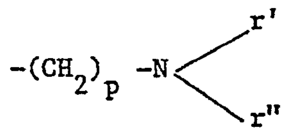

p being 1, 2 or 3, r′ and r″ representing a hydrogen atom, a lower alkyl radical, a monohydroxyalkyl or polyhydroxyalkyl radical, an optionally substituted aryl radical, an amino acid residue or an amino sugar residue, or alternatively, taken together, form a heterocycle, X represents an oxygen atom, a sulphur atom, SO, $SO_2$ or a radical $-NR_4$, and Y represents $CR_4$ or a nitrogen atom

$R_4$ representing a hydrogen atom or a lower alkyl radical, and the salts of the said heterocyclic derivatives of formula (I), characterized in that it consists in reacting an aromatic carboxylic acid derivative of formula:

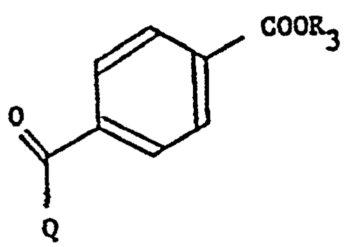

in which:

Q represents OH or Cl and $R_3$ is as defined above with the exception of a hydrogen atom,

(a) either with a diamino, hydroxyamino or thioamino aromatic derivative of formula:

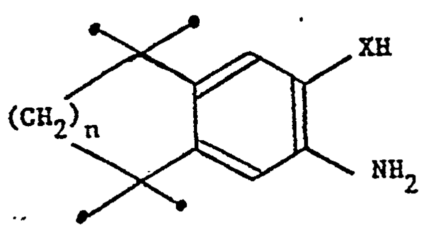

in which

n is 1 or 2 and X represents NH, O or S and in performing a dehydrating cyclization reaction in the presence of an acid catalyst,

(b) or with a derivative of fomula:

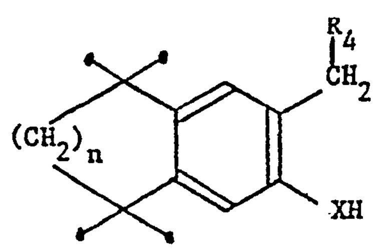

in which:

n is 1 or 2, $R_4$ is as defined above and X represents NH, O or S, and in performing a bromination of the compound obtained and then in cyclizing the intermediate derivative bearing a phosphonium or phosphinyl group according to the conditions of the Wittig or Wittig-Honer reactiion and, where appropriate, in

converting the said compounds obtained, according to known methods, for the purpose of obtaining any one of the other meanings of the radical $R_1$.

2. Process according to Claim 1, characterized in that the dehydrating cyclization reaction is performed in the presence of a sulphonic acid such as p-toluene-sulphonic acid in an inert solvent such as toluene or xylene.

3. Process according to Claim 1, characterized in that the bromination reaction is performed in the presence of N-bromosuccinimide in benzene or carbon tetrachloride.

4. Cosmetic composition for body and hair hygiene, characterized in that it contains at least one compound of formula (I) according to Claim 1, in a suitable cosmetic vehicle.

5. Cosmetic composition according to Claim 4, characterized in that it contains the compound of formula (I) at a concentration of between 0.00001 and 0.01% by weight.